# EUROPEAN PATENT APPLICATION

(11) **EP 1 548 108 A1**
(43) Date of publication of application: **29.06.2005**
(21) Application number: 03794242.2
(22) Date of filing: 05.09.2003
(51) Int. Cl.: C12N 15/09, C12Q 1/02, C12N 1/19

(54) **METHOD OF SCREENING PHYSIOLOGICALLY ACTIVE SUBSTANCE**

(30) Priority: 05.09.2002 JP 2002260515; 26.09.2002 JP 2002280512
(71) Applicant: Epoch Medical International Inc., Osaka-shi, Osaka 541-0046 (JP)
(72) Inventor: Katsuyama, I., Epoch Medical International inc., Osaka-shi, Osaka 541-0046 (JP); Arakawa, T., Alliance Protein Laboratories, Camarillo, CA 93012 (US); Tokunaga, M., Fac. of Agriculture, Kagoshima Univ., Kagoshima 890-0065 (JP); Yamamoto, T., Institute of Medical, Science, Minato-ku, Tokyo 108-0071 (JP)
(74) Representative: VOSSIUS & PARTNER
(86) International application number: PCT/JP2003/011329
(87) International publication number: WO 2004/022742

(57) **Abstract**

The present invention relates to a method of screening a physiologically active substance, comprising the steps of (1) contacting a transformed yeast with a test sample, wherein the transformed yeast is capable of expressing a heterogeneous protein, and shows a change in a growing state in an expression state of the protein as compared to that in a non-expression state of the protein; (2) culturing the yeast under conditions that the protein is capable of being expressed; and (3) measuring the growing state of the yeast, wherein the physiologically active substance is judged to be present in the test sample in a case where the growth of the yeast is lowered or improved in the presence of the test sample as compared to that in the absence of the test sample as a control; an yeast to be used in the method; a physiologically active substance obtainable by the method of screening as defined above; and a kit for screening a physiologically active substance, comprising the yeast as defined above.

## Description

### TECHNICAL FIELD

The present invention relates to a method of screening a physiologically active substance which is capable of acting on proliferation and differentiation of a cell.

### BACKGROUND ART

So far, various factors involved in regulating cell cycle have been reported, and function thereof has gradually been revealed. For instance, a group of proteins referred to as Tob family have been known. As a gene encoding the proteins, six kinds of members have been found from mammals to date (see, for instance, Yutaka Yoshida, Tadashi Yamamoto (2001) *Jikken Igaku (Experimental Medicine),* **19**, 1194-1198). For instance, it has been reported that when Tob is forcibly expressed in cultured cells, cell proliferation suppression is exhibited (see, for instance, Suzuki, T., K-Tsuzuku, J., Ajima, R., Nakamura, T., Yoshida, Y., and Yamamoto, T,. (2002) *Genes Dev*., **16**, 1356-1370). Although accurate molecular mechanism of this cell proliferation suppression is unknown, it is thought that Tob is involved in both arrests of G0/G1 phase and G2/M phase of cell cycle, suggesting that the proteins function as a cofactor for transcription factors. In addition, it has been known that a bone mass is increased in a tob gene-deficient mouse (see, for instance, Yoshida, Y., Tanaka, S., Umemori, H., Minowa, O., Usui, M., Ikematsu, N., Hosoda, E., Imamura, T., Kuno, J., Yamashita, T., Miyazono, K., Noda, M., Noda, T., and Yamamoto, T. (2000) Cell, 103, 1085-1097). It has been clarified that Tob binds to an intracellular signaling molecule Smad to suppress a bone morphogenetic protein factor (BMP) signal, and suppresses BMP-dependent proliferation and differentiation of osteoblast to control osteogenesis (see, for instance, Yoshida, Y., Tanaka, S., Umemori, H., Minowa, O., Usui, M., Ikematsu, N., Hosoda, E., Imamura, T., Kuno, J., Yamashita, T., Miyazono, K., Noda, M., Noda, T., and Yamamoto, T. (2000) *Cell,* **103**, 1085-1097).

In addition, a group of proteins referred to as Caf family have been known. The proteins are present in all eukaryotes including yeast. For instance, it has been reported that Caf1 influences the cell cycle by binding to CCR (carbon catabolite repressor) 4 (see, for instance, Chen, J., Rappsilber, J., Chiang, Y. C., Russell, P., Mann, M., Denis, C. L., *J. Mol. Biol. (2001)* **314**, 683-94 and Bai, Y., Salvadore, C., Chiang, Y. C., Collart, M. A., Liu, H. Y., and Denis, C. L., *Mol. Cell. Biol.* (1999) **19**, 6642-6651). In addition, it has been known that Caf1 interacts with Tob, and there is thought to be a possibility that the interaction of the two factors causes brake in transition from G0 phase to G1 phase in the cell cycle. It has been reported that incidence of cancer is significantly increased in a mouse in which *tob* gene has been knocked out (see, for instance, JP 2001-211782A (page 2)).

On the other hand, as a technique of using yeast deficient in aspiration ability, for instance, a process of producing a protein using a transformant of an yeast transformed with a DNA comprising a gene of a heterogeneous protein has been known (see, for instance, JP-A-Hei 2-418 (claims, page 2)). In the publication, it is disclosed that expression of the gene is enhanced by making aspiration ability deficient in an yeast used in the production of a protein.

However, the publication does not disclose that yeast deficient in aspiration ability can be used for screening a physiologically active substance, or that efficiency of the screening can be enhanced thereby.

### DISCLOSURE OF INVENTION

There is a possibility that a physiologically active substance having a similar function to the factors involved in regulating cell cycle as described above, especially factors involved in intracellular signaling at a G0/G1 phase of a mammal, such as Tob family proteins and Caf family proteins, or those having a function of inhibiting or enhancing the function may serve as a prophylactic agent and/or therapeutic agent for various diseases involved in proliferation or differentiation of cells. For instance, there is a possibility that a substance inhibiting the function of Tob may serve as a prophylactic agent and/or therapeutic agent for bone diseases such as osteoporosis and fracture, and there is a possibility that a substance exhibiting a similar action to that of Tob by interaction with Caf1 serves as a prophylactic agent for carcinogenesis and/or an anti-cancer agent.

Accordingly, an object of the present invention is to provide a method of screening a physiologically active substance using yeast, capable of efficiently screening a physiologically active substance. Also, an object of the present invention is to provide an yeast used in the method, a physiologically active substance obtainable by the screening method, and a kit for screening a physiologically active substance comprising the yeast.

Specifically, the present invention relates to:
[1] a method of screening a physiologically active substance, comprising the steps of:
   (1) contacting a transformed yeast with a test sample, wherein the transformed yeast is capable of expressing a heterogeneous protein, and shows a change in a growing state in an expression state of the protein as compared to that in a non-expression state of the protein;
   (2) culturing the yeast under conditions that the protein is capable of being expressed; and
   (3) measuring the growing state of the yeast,
   wherein the physiologically active substance is judged to be present in the test sample in a case where the growth of the yeast is lowered or improved in the presence of the test sample as compared to that in the absence of the test sample as a control;
[2] the method of screening according to the above [1], wherein the heterogeneous protein is capable of lowering the growth of the yeast as compared to that of the non-expression state;
[3] the method of screening according to the above [1] or [2], wherein the heterogeneous protein is a protein involved in regulating cell cycle of a mammal cell;
[4] the method of screening according to the above [3], wherein the protein involved in regulating cell cycle of a mammal cell is a protein involved in intracellular signaling of G0/G1 phase of a mammal cell;
[5] the method of screening according to any one of the above [1] to [4], wherein the growing state of the yeast is determined in the step (3) by monitoring a change in turbidity of an yeast culture medium, a morphological change of the yeast, a change in wet-weight of the yeast, a change in dry-weight of the yeast, a change in endogenous enzyme activity of the yeast or a change in amount of an endogenous protein of the yeast;
[6] the method of screening according to any one of the above [1] to [5], wherein the transformed yeast is deficient in aspiration ability;
[7] a transformed yeast being capable of expressing a heterogeneous protein, and showing a change in a growing state in an expression state of the protein as compared to that in a non-expression state of the protein;
[8] the transformed yeast according to the above [7], wherein the change in the growing state is lowering of the growth;
[9] the transformed yeast according to the above [7] or [8], wherein the heterogeneous protein comprises at least an active site of the protein in an active state;
[10] the transformed yeast according to any one of the above [7] to [9], wherein the heterogeneous protein is a protein involved in regulating cell cycle of a mammal cell;
[11] the transformed yeast according to the above [10], wherein the protein involved in regulating cell cycle of a mammal cell is a protein involved in intracellular signaling of G0/G1 phase of a mammal cell;
[12] the transformed yeast according to the above [11], wherein the protein involved in intracellular signaling of G0/G1 phase of a mammal cell is a protein belonging to Tob family and/or a protein belonging to Caf family;
[13] the transformed yeast according to the above [12], wherein the protein belonging to Tob family is:
   (a) a protein comprising in an N-terminal region of the amino acid sequence thereof the amino acid sequence of SEQ ID NO: 1;
   (b) a protein comprising in an N-terminal region of the amino acid sequence thereof an amino acid sequence having deletion, addition, insertion or substitution of at least one amino acid residue in the amino acid sequence of SEQ ID NO: 1, wherein the protein induces growth inhibition of the transformed yeast in the expression state;
   (c) a protein comprising in an N-terminal region of the amino acid sequence thereof an amino acid sequence having a sequence identity of 20% or more to the amino acid sequence of SEQ ID NO: 1, wherein the protein induces growth inhibition of the transformed yeast in the expression state; or
   (d) a protein comprising in an N-terminal region of the amino acid sequence thereof an amino acid sequence having a sequence identity of 20% or more to the amino acid sequence of SEQ ID NO: 2 in a region from an N-terminal to 100 amino acid residues, wherein the protein induces growth inhibition of the transformed yeast in the expression state;
[14] the transformed yeast according to the above [12], wherein the protein belonging to Caf family is:
   (a) a protein comprising the amino acid sequence of SEQ ID NO: 4;
   (b) a protein comprising an amino acid sequence having deletion, addition, insertion or substitution of at least one amino acid residue in the amino acid sequence of SEQ ID NO: 4, wherein the protein induces growth inhibition of the transformed yeast in the expression state; or
   (c) a protein comprising an amino acid sequence having a sequence identity of 20% or more to the amino acid sequence of SEQ ID NO: 4, wherein the protein induces growth inhibition of the transformed yeast in the expression state.
[15] the transformed yeast according to any one of the above [7] to [14], wherein the transformed yeast comprising a gene encoding a heterogeneous protein operably in expression ligated to a promoter;
[16] the transformed yeast according to any one of the above [7] to [15], wherein the transformed yeast is deficient of aspiration ability, which is used for the method of screening as defined in the above [6];
[17] a physiologically active substance obtainable by the method of screening as defined in any one of the above [1] to [6]; and
[18] a kit for screening a physiologically active substance, comprising the yeast as defined in any one of the above [7] to [16].

### BRIEF DESCRIPTION OF THE DRAWINGS

Figure 1 is a diagram showing a proliferation curve of a transformant of an YPH500α strain into which *tob* gene is introduced.
Figure 2 is a diagram showing the results of Western blotting of a culture of a transformant of an YPH500α strain into which *tob* gene is introduced at a point where the cells are cultured for 9 hours.
Figure 3 is a diagram showing the results of Western blotting of a culture of a transformant of an YPH500α strain into which *tob* gene is introduced at a point where the cells are cultured for 78 hours.
Figure 4 is a diagram showing the results of Western blotting of a culture of a transformant of an YPH500α strain into which *tob* gene is introduced at a point where the cells are cultured for 181 hours.
Figure 5 is a diagram showing a proliferation curve of a transformant of an YPH500α strain into which *tob* gene is introduced.
Figure 6 is a diagram showing a proliferation curve of a transformant of a 20B-12 strain into which *tob* gene is introduced.
Figure 7 is a diagram showing the results of Western blotting of a culture of a transformant of an YNN27 strain into which *tob* gene is introduced.
Figure 8 is a diagram showing the results of Western blotting of a culture of a transformant of a 20B-12 strain into which *tob* gene is introduced.
Figure 9 is an agarose gel electrophoretic diagram showing the results for confirming the presence of pESC-TRP in the transformant of an YPH500α strain.
Figure 10 is an agarose gel electrophoretic diagram showing the results for confirming the presence of pESC-TRP-*tob* in the transformant of an YPH500α strain.
Figure 11 is a diagram showing a proliferation curve of a transformant of an YPH500α strain into which *hcaf1* gene is introduced.
Figure 12 is a diagram showing the results of Western blotting of a culture of a transformant of an YPH500α strain into which *hcaf1* gene is introduced.
Figure 13 is a diagram showing a proliferation curve of a transformant of an INVSc1 strain into which *hcaf1* gene is introduced.
Figure 14 is a diagram showing the results of Western blotting of a culture of a transformant of an INVSc1 strain into which *hcaf1* gene is introduced.
Figure 15 is a diagram showing a proliferation curve of a transformant of an INVSc1 strain into which *tob* gene and *hcaf1* gene are introduced.
Figure 16 is a diagram showing the results of Western blotting of a culture of a transformant of an INVSc1 strain into which *tob* gene and *hcaf1* gene are introduced.
Figure 17 is a diagram showing a proliferation curve of a transformant of an YPH500-12 strain (ρ⁻) into which *tob* gene is introduced.
Figure 18 is a diagram showing the results of Western blotting of a culture of a transformant of an YPH500-12 strain (ρ⁻) into which *tob* gene is introduced at a point where the cells are cultured for 69 hours.
Figure 19 is a diagram showing a proliferation curve of a transformant of an YPH500-12 strain (ρ⁻) into which *hcaf1* gene is introduced.
Figure 20 is a diagram showing the results of Western blotting of a culture of a transformant of an YPH500-12 strain (ρ⁻) into which *hcaf1* gene is introduced at a point where the cells are cultured for 45 hours.
Figure 21 is a diagram showing a proliferation curve of a transformant of an YPH500-12 strain (ρ⁻) into which *tob* gene and *hcaf1* gene are introduced.
Figure 22 is a diagram showing the results of Western blotting of a culture of a transformant of an YPH500-12 strain (ρ⁻) into which *tob* gene and *hcaf1* gene are introduced at a point where the cells are cultured for 144 hours.
Figure 23 is a diagram showing a proliferation curve of a transformant of an YPH500-12 strain (ρ⁻) into which *lck* gene is introduced.
Figure 24 is a diagram showing the results of Western blotting of a culture of a transformant of an YPH500-12 strain (ρ⁻) into which *lck* gene is introduced at a point where the cells are cultured for 47 hours.
Figure 25 is a diagram showing a proliferation curve of a transformant of an YPH500-12 strain (ρ⁻) into which *lckYF* gene is introduced.
Figure 26 is a diagram showing the results of Western blotting of a culture of a transformant of an YPH500-12 strain (ρ⁻) into which *lckYF* gene is introduced at a point where the cells are cultured for 95 hours.
Figure 27 is a diagram showing a proliferation curve of a transformant of an YPH500-12 strain (ρ⁻) into which *tob* gene is introduced by simultaneously using four kinds of vectors.
Figure 28 is a diagram showing a proliferation curve of a transformant of an YPH500-12 strain (ρ⁻) into which *tob* gene is introduced.
Figure 29 is a diagram showing the results of Western blotting using a Tob antibody, of a culture of a transformant of an YPH500-12 strain (ρ⁻) into which *tob* gene is introduced by simultaneously using four kinds of vectors at a point where the cells are cultured for 168 hours.
Figure 30 is a diagram showing the results of Western blotting using a myc antibody, of a culture of a transformant of an YPH500-12 strain (ρ⁻) into which *tob* gene is introduced by simultaneously using four kinds of vectors at a point where the cells are cultured for 168 hours.

### BEST MODE FOR CARRYING OUT THE INVENTION

In the method of screening a physiologically active substance of the present invention, a transformed yeast capable of expressing a heterogeneous protein is cultured under conditions capable of expressing the heterogeneous protein, that is, under conditions that is capable of changing the growing state of the yeast, the yeast is contacted with a test sample during the culture, wherein the physiologically active substance is judged to be present in the test sample in a case where the growth of the yeast is lowered or improved when the test sample is present (in the presence) as compared to that when the test sample is absent (in the absence) as a control.

The term "heterogeneous protein" as used herein refers to a heterogeneous protein for an yeast capable of inducing a change in the growing state of an yeast. The "heterogeneous protein" includes a fragment of the protein, as long as the fragment has a similar function to that of the protein. In addition, the phrase "capable of expressing a heterogeneous protein" refers to that an yeast comprises a gene encoding a heterogeneous protein (in other words, an yeast being transformed with the gene), wherein the protein can be actually expressed. In addition, the term "physiologically active substance" refers to a substance capable of exhibiting some sort of physiological actions on a living body, for instance, a substance capable of acting on proliferation or differentiation of cells, in particular, a compound capable of influencing intracellular signaling at a G0/G1 phase of mammal cells, for instance, a compound having a similar function to that of a factor involved in intracellular signaling at a G0/G1 phase of mammal cells or a function of inhibiting or enhancing the function.

In the method of screening a physiologically active substance of the present invention, the influence of a test sample on the growing state of an yeast in the presence of the test sample using the absence of the test sample as a control can be accurately grasped by preparing each of proliferation curves of an yeast expressing a heterogeneous protein in the absence and the presence of the test sample, and comparing the obtained proliferation curves. Therefore, the presence of a physiologically active substance in the test sample can be detected with high reliability. Therefore, according to the screening method of the present invention, a physiologically active substance can be efficiently screened.

The yeast used in the present invention is capable of expressing a heterogeneous protein. The yeast is encompassed in the present invention. The yeast has the properties that a change in the growing state, for instance, lowering or improvement in growth, is found in an expression state (in the expression state), as compared to the state in which the heterogeneous protein is not expressed (in the non-expression state). In addition, it is preferable that the yeast used in the present invention is deficient in aspiration ability. In other words, the yeast deficient in aspiration ability (i) is more sensitive in a change in the growing state, and (ii) shows more stable growth in a non-expression state of a heterogeneous protein, as compared to those of an yeast capable of expressing a heterogeneous protein and having aspiration ability. The phenomenon as described above is deduced to be associated with deficiency in aspiration ability of the yeast, but the detail of the mechanism thereof is unknown. However, taking into consideration the fact that the above (ii) is found, it is considered that the above (i) does not simply depend upon, for instance, increase in expression level of a heterogeneous protein accompanied with deficiency in aspiration ability (for instance, JP-A-Hei 2-418).

The term "lowering in growth" refers to the fact that a generation time of the yeast is longer in the expression state as compared to that in the non-expression state, when the proliferation curves of the yeast in a non-expression state and in an expression state of a heterogeneous protein are obtained, and the respective generation time is obtained and compared. Alternatively, a proliferation rate may be obtained in place of the generation time. In this case, the lowering in growth refers to lowering in the slope of a proliferation curve in a logarithmic proliferation phase. On the other hand, the term "improvement in growth" refers to the fact that the generation time of the yeast is shorter in the expression state as compared to that in the non-expression state. Alternatively, the improvement in growth refers to increase in the slope of a proliferation curve in a logarithmic proliferation phase. The phrase "more sensitive in a change in the growing state" refers to the fact that a uniform change in the growing state is found without variance between yeast individuals expressing the same kind of heterogeneous protein. The term "stable growth" refers to the fact that a uniform growing state is shown without variance between yeast individuals in the non-expression state of the same kind of the heterogeneous protein.

The proliferation curve is prepared by plotting values of an arbitrary parameter showing an extent of proliferation of the yeast against culture time (for instance, turbidity of an yeast culture medium described later). A specific method of evaluating the properties of the yeast of the present invention will be described later.

The heterogeneous protein is not particularly limited as long as the protein is one that is capable of inducing a change in the growing state of the yeast. The protein is preferably a protein which is capable of lowering or improving the growth of an yeast, more preferably a protein which is capable of lowering the growth of an yeast. Alternatively, the protein is preferably a protein involved in proliferation or differentiation of cells, more preferably a protein involved in regulating cell cycle of a mammal cell. Among them, the protein involved in intracellular signaling at a G0/G1 phase of a mammal cell is especially preferable.

The phrase "protein involved in intracellular signaling at a G0/G1 phase of mammal cells (hereinafter, referred to as signal protein)" as used herein refers to a protein involved in processes from G0 phase to G1 phase, and from G1 phase to S phase in the cell cycle in a mammal cell, wherein the protein has the function of controlling progression or arrest thereof. In addition, the term "mammal cell" may be any cell derived from a mammal, and the mammal cell includes, for instance, cells derived from Primates such as human and monkey, Rodentia such as mouse, rat and guinea pig, Artiodactyla such as bovine and swine, Carnivora such as cat, Proboscidea such as elephant, Perissodactyla such as horse, Lagomorpha such as rabbit, Chiroptera such as bat, Marsupialia such as kangaroo and wallaby, Monotremat such as platypus and echidna, and the like.

It has been considered that a signal protein generally has the property of lowering the growth of an yeast when the protein is expressed in the yeast. The signal protein includes, for instance, proteins belonging to Tob family, proteins belonging to Caf family, proteins belonging to cyclin family, proteins belonging to CDK (cyclin-dependent kinase) family, proteins belonging to Rb (retinoblastoma) family gene product, proteins belonging to p53 family gene product, proteins belonging to p21 family gene product, proteins belonging to GADD45 (growth arrest and DNA damage inducible) family gene product, proteins belonging to E2F family proteins, proteins belonging to p15 family gene product, proteins belonging to p16 family gene product, proteins belonging to Arf family gene product, proteins belonging to INK4 family gene product, proteins belonging to TGFβ family, histone diesterases and the like.

The protein belonging to Tob family usually comprises a homologous region consisting of about 110 amino acid residues having homology with the amino acid sequence shown in SEQ ID NO: 1 in an N-terminal region of the amino acid sequence thereof. Therefore, as the protein, ① a protein comprising the amino acid sequence shown in SEQ ID NO: 1 in the N-terminal region, or ② a protein having the homologous region is preferable. The above-mentioned protein ② may be a protein comprising an amino acid sequence having deletion, addition, insertion or substitution of at least one amino acid residue in the amino acid sequence of SEQ ID NO: 1 in an N-terminal region thereof, or the protein may be a protein comprising an amino acid sequence having a sequence identity of preferably 20% or more, more preferably 35% or more, even more preferably 50% or more, even more preferably 65% or more, even more preferably 80% or more, especially preferably 90% or more, most preferably 95% or more, to the amino acid sequence of SEQ ID NO: 1, as long as the protein induces the lowering in the growth of an yeast in the expression state thereof.

Further, it has been known that an even more highly homologous region (highly conserved region) referred to as Box A (also referred to as GR Box) consisting of the amino acid sequence shown in SEQ ID NO: 2 is present in a region from an N-terminal to 100 amino acid residues in the above-mentioned homologous region of the protein belonging to Tob family. In addition, it has been known that a highly conservative region referred to as Box B consisting of the amino acid sequence shown in SEQ ID NO: 3 in addition to the Box A may be present in the above-mentioned homologous region in some proteins belonging to Tob family. For Box A and Box B, a reference is made to Tirone, F., (2001) *J. Cell Physiol.,* **187,** 155-165. For instance, as to proteins belonging to Tob family for which the presence of Box A and Box B has been known, positions of the boxes on the amino acid sequence are concretely shown as follows: hTob has Box A at 40th to 58th positions from the N-terminal, and Box B at 76th to 95th positions from the N-terminal; hTob2 has Box A at 40th to 58th positions from the N-terminal, and Box B at 76th to 96th positions from the N-terminal; Pc3 has Box A at 50th to 68th positions from the N-terminal, and Box B at 124th to 143rd positions from the N-terminal; hAna has Box A at 42nd to 60th positions from the N-terminal, and Box B at the 88th to 117th position from the N-terminal; mBtg3 has Box A at 42nd to 60th positions from the N-terminal, and Box B at 88th to 117th positions from the N-terminal; and hPc3B has Box A at 42nd to 60th positions from the N-terminal, and Box B at 88th to 117th positions from the N-terminal. Therefore, the protein belonging to Tob family may be a protein comprising an amino acid sequence having a sequence identity of preferably 20% or more, more preferably 35% or more, even more preferably 50% or more, even more preferably 65% or more, even more preferably 80% or more, especially preferably 90% or more, most preferably 95% or more, to the amino acid sequence of SEQ ID NO: 2 in a region from the N-terminal to 100 amino acid residues, as long as the protein is capable of inducing lowering in the growth of an yeast in the expression state thereof.

The protein belonging to Tob family is preferably a protein having both of Box A and Box B.

The protein belonging to Caf family is represented by hCaf1. hCaf1 is a protein consisting of the amino acid sequence shown in SEQ ID NO: 4. The protein belonging to Caf family may be a protein comprising an amino acid sequence having deletion, addition, insertion or substitution of at least one amino acid residue in the amino acid sequence of SEQ ID NO: 4, or a protein comprising an amino acid sequence having a sequence identity of preferably 20% or more, more preferably 35% or more, even more preferably 50% or more, even more preferably 65% or more, even more preferably 80% or more, especially preferably 90% or more, most preferably 95% or more, to the amino acid sequence of SEQ ID NO: 4, as long as the protein is capable of inducing lowering in the growth of an yeast in the expression state thereof.

The above-mentioned phrase "at least one" means one or several. In addition, the term "N-terminal region" as used herein refers to a region beginning from the N-terminal and up to 400 residues from the N-terminal in the amino acid sequence.

The sequence identity of the amino acid sequences refers to sequence similarity for amino acid residues between the two amino acid sequences. The sequence identity can be determined by comparing two sequences which are aligned in the optimal state over a region of amino acid sequences to be compared.

The numerical value (percentage) of the sequence identity is calculated by determining the identical amino acid residues present in both the sequences to determine the number of matched sites, thereafter dividing the above-mentioned number of the matched sites by a total number of amino acid residues within the sequence regions to be compared, and multiplying the resulting numerical value by 100. The sequence identity can be obtained by utilizing sequence analysis software, for instance, network services such as BLASTP and FASTA.

For instance, the protein belonging to Tob family includes, for instance, Tob, Tob2, Btg1, Pc3/Tis21/Btg2, Ana/Btg3, B9.10, Pc3K, Pc3B, B9.15 and the like. The protein belonging to Caf family includes Caf1, Caf2, POP2 and the like. In addition, the protein belonging to cyclin family includes, for instance, cyclin A, cyclin B, cyclin D1, D2, D3, cyclin E and the like.

As the yeast of the present invention, an yeast capable of expressing a heterogeneous protein (or fragment thereof) comprising at least an active site in the active state is preferable, from the viewpoint of exhibition of the desired effects of the present invention. A heterogeneous protein as a so-called complete protein which is not a fragment thereof as used herein refers to a complete protein consisting of a full length of an amino acid sequence thereof, or a substantially complete protein having deletion, addition or the like of one or several amino acid residues. On the other hand, a fragment thereof refers to a part of the heterogeneous protein as a complete protein. The fragment is suitably a peptide consisting of amino acids of preferably 5 residues or more, more preferably from 5 to 100,000 residues, even more preferably from 10 to 50,000 residues, especially preferably from 100 to 10,000 residues. The fragment thereof includes, for instance, a peptide comprising an active site of a signal protein and consisting of amino acids of preferably 5 residues or more, more preferably from 5 to 100,000 residues, even more preferably from 10 to 50,000 residues, especially preferably from 100 to 10,000 residues.

The term "active site" refers to a site that is necessary for exhibiting the activity of the heterogeneous protein. For instance, in a signaling protein, an active site corresponds to a site necessary for exhibiting the function involved in intracellular signaling at a G0/G1 phase of cell cycle. Although the position of an active site of a signaling protein on an amino acid sequence and an amino acid sequence constituting the site are still unknown, it is deduced that an active site of Tob is present in the above-mentioned homologous region. An amino acid sequence of a homologous region which is deduced to constitute an active site Tob is shown in SEQ ID NO: 1.

In addition, the phrase "comprises ... in the active state" refers to one in which an active site has a higher order structure necessary for expression of activity. For instance, in the case of the signaling protein or a fragment thereof, whether or not the signal protein or a fragment thereof comprises an active site in the active state can be evaluated by whether or not the lowering in the growth of the yeast is caused in the expression state thereof. In other words, when the lowering in the growth of the yeast is caused, it is judged that the expressed signaling protein or a fragment thereof comprises an active site in the active state.

The yeast of the present invention is preferably an yeast capable of expressing a protein belonging to Tob family and/or a protein belonging to Caf family. Among them, an yeast capable of coexpressing both the protein belonging to Tob family and the protein belonging to Caf family is more preferable. For instance, when Tob and Caf1 are coexpressed in an yeast, lowering in growth of yeast is enhanced as compared to the case where each of the proteins is expressed alone. Therefore, it is considered that lowering in the growth of an yeast can be more clearly captured by coexpressing both the protein belonging to Tob family and the protein belonging to Caf family. More concretely, an yeast capable of expressing Tob, Tob2, Btg1, Pc3/Tis21/Btg2, Ana/Btg3, B9.10, Pc3K, Pc3B, or B9.15 as the protein of Tob family; an yeast capable of expressing Caf1, Caf2, or POP2 as the protein of Caf family; or an yeast capable of expressing a combination of at least one each of those proteins concretely shown herein as the combination of the protein of Tob family and the protein of Caf family is especially preferable, from the viewpoint of exhibiting the desired effects of the present invention.

The yeast deficient in aspiration ability suitably used in the present invention can be obtained by (i) transforming an yeast with a gene encoding a heterogeneous protein (or a fragment thereof), and obtaining a strain deficient in aspiration ability of the resulting yeast, or (ii) obtaining a strain deficient in aspiration ability, and thereafter transforming the strain with a gene encoding a heterogeneous protein (or a fragment thereof). The yeast having aspiration ability can be obtained in the same manner with a usual yeast described later except that the step of making an yeast deficient in aspiration ability is not carried out.

It is preferable that the transformation of an yeast with a gene encoding a heterogeneous protein (or a fragment thereof) is carried out by introducing a vector, preferably an expression vector, comprising the gene into the yeast.

The expression vector usually comprises a promoter, a gene encoding a heterogeneous protein and a terminator. The gene is operably in expression ligated to a promoter. The phrase "operably in expression ligated to a promoter" as used herein refers to ligation of the gene to the promoter in such a manner that expression of the protein encoded by the gene can be induced under the control of the promoter. In many cases, the expression vector contains an antibiotic resistance gene, an auxotrophic gene, and an origin of replication which functions in *Escherichia coli.* Further, an enhancer and an origin of replication which functions in yeast may be contained. The arrangement of these elements constituting the expression vector can be appropriately determined so as to exhibit the desired effects.

The above-mentioned expression vector can be constructed by introducing a promoter, a multicloning site, a terminator and the like in this order into a given plasmid vector of YIp type, YEp type, YRp type or YCp type known as an yeast vector, and introducing into the multicloning site a gene encoding a heterogeneous protein in which a translation initiation codon is added to upstream of the gene, and a translation termination codon is added to downstream of the gene. In the construction of the recombinant expression vector as mentioned above, the conventional recombinant DNA technique can be utilized (refer to, for instance, Sambrook et al., *Molecular Cloning : A* *Laboratory Manual 2*^{*nd*} *ed.,* Cold Spring Harbor Laboratory Press, 1989, and the like). As the above-mentioned plasmid vector, the YEp type plasmid vector is desirable from the viewpoint that one having a higher expression level of a heterogeneous protein, for instance, a signal protein, is preferable. Alternatively, the YCp type plasmid vector is desirable from the viewpoint of high stability of retaining a plasmid vector in a host. The vector includes, for instance, YEp24, YEp13, and the like.

As the above-mentioned promoter, for instance, an inducible promoter such as *AOX1, GAL1, GAL7, GAL10, LAC4, PHOS,* or *SUC2* is suitably used. Alternatively, a non-inducible promoter of a glycolysis enzyme such asADH or *PGK* may be used. The terminator may be arbitrarily selected depending upon the promoter to be used. The antibiotic resistance gene used as a selectable marker gene includes, for instance, an ampicillin resistance gene and a tetracycline resistance gene for *Escherichia coli,* a G418 resistance gene for an yeast and the like. In addition, the gene compensating for auxotrophic mutation includes *LEU2, URA3, TRP1, HIS4, ADE2* and the like.

In addition, in the construction of the expression vector in the present invention, various vectors into which a promoter such as GAL1 is previously incorporated may be utilized. The vector includes, for instance, pESC Yeast Epitope Tagging Vectors (manufactured by Stratagene) and the like. Further, in the case where it is desired that a heterogeneous protein expressed in an yeast is tagged, the above-mentioned pESC Yeast Epitope Tagging Vectors may be used, since the vectors are tagged. In this case, there is an advantage that a desired protein can be detected and purified with an antibody specific to a tag or affinity specific to a tag.

The information on the nucleotide sequence of a gene encoding a desired heterogeneous protein can be obtained, for instance, from GenBank. Examples of the gene encoding a signal protein as preferred examples of a heterogeneous protein are as follows: For instance, as to the genes respectively encoding the protein belonging to Tob family and the protein belonging to Caf family, the names of each gene and corresponding accession numbers thereof are exemplified hereinbelow: *tob* (Accession No.: D38305 (human); D78382 (mouse)), *tob2* (Accession No.: AB035207 (human); AB041225 (mouse)), *btg1* (Accession No.: X61123 (human); L16846 (mouse); X64146 *(G. domesticus)), pc3*/*tis21*/*btg2* (Accession No.: M60921 (rat); M64292 (mouse); Y09943 (human)), *ana*/*btg3* (Accession No.: NM006806.1 (human); Z72000 (mouse)), *b9.10* (Accession No.: X73316 *(X. leevis)), pc3B* (Accession No.: AJ271351.1 (human); AJ005120 (mouse)), *b9.15* (Accession No.: X73317 (*X. leevis*)), *caf1* (Accession No.: L46722 (human); U21855 (mouse)), *pop2* (Accession No.: D12807 *(Saccharomyces cerevisiae*)). In addition, as to *pc3K* and *caf2,* references are to be made to Prevot, D., Morrel, A-P., Voeltzel, T., Rostan, M-C., Rimokh, R., Magaud, J-P., and Corbo, L., *(2001) J. Biol. Chem.,* **276,** 9640-9648 and Beko, Z., Sopiczki, M., and Carr, A.M.(1998) *Mol. Gen. Genet.,* **260,** 434-443.

The gene encoding a heterogeneous protein, for instance, a signal protein, or a fragment thereof can be properly amplified by preparing one pair of primers based on the information on the nucleotide sequence obtained, and performing polymerase chain reaction (PCR) using, for instance, a genomic DNA or a cDNA library of human as a template DNA.

For instance, when, it is desired to express a protein having an amino acid sequence having deletion, addition, insertion or substitution of at least one amino acid residue in the above-mentioned specified amino acid sequence as the protein belonging to Tob family or Caf family, a desired mutation is introduced into, for instance, a gene encoding the amino acid sequence by a known random mutagenesis method or site-directed mutagenesis method, and the resulting gene may be used as a gene to be introduced into an yeast. In addition, for instance, when a fragment of a signal protein is desired to be expressed, for instance, PCR is performed by properly selecting a sequence of a primer so that only a gene region encoding a desired fragment is amplified, and the resulting gene may be used as a gene to be introduced into an yeast. Alternatively, the fragment can be also expressed by a method of expressing the fragment comprising introducing a stop codon into a given position of a gene encoding a signal protein, or a method of expressing the fragment comprising cleaving a gene region corresponding to the desired partial peptide with a restriction enzyme, and introducing the resulting fragment into an appropriate expression vector.

Usually, the transformation of the yeast may be carried out with one kind of an expression vector comprising a gene encoding one kind of a heterogeneous protein. For instance, an yeast is transformed with one kind of an expression vector containing *tob* or *caf1*. On the other hand, an yeast may be transformed with two or more kinds of expression vectors, each comprising a gene encoding one kind of a heterogeneous protein, or with one kind of an expression vector comprising a gene encoding two or more kinds of heterogeneous proteins. For instance, the yeast is transformed using one kind of an expression vector containing *tob* and one kind of an expression vector comprising *caf1,* or with one kind of an expression vector comprising a gene portion in which *tob* and *caf1* are indirectly ligated (hereinafter, referred to as tandem expression vector).

The tandem expression vector is obtained, for instance, in the manner as described below. For instance, in a single expression vector, a gene such as *tob* and a promoter are arranged in the vector so that each of the transcriptions of *tob* and *caf1* can be controlled under the controls of different promoters. The promoters and other elements which can be used in these methods are the same as those described above.

In addition, a gene encoding a heterogeneous protein may be inserted into chromosome of an yeast by homologous recombination. For instance, an yeast may be transformed with a vector comprising a gene, for instance, *tob* or the like which is positioned between flanking sequences comprising nucleotides sequences having homology to two nucleotide sequences respectively on both sides of a desired target insertion site of the gene on the chromosome. As the target insertion site, preferable is a site in which an endogenous promoter of an yeast is capable of being operably in expression ligated to a gene such as *tob* by inserting the gene into the site. In this case, since a gene such as *tob* can be expressed under the control of the endogenous promoter of an yeast, it is not necessarily required that the gene is operably in expression ligated to a promoter in a vector used for the transformation. On the other hand, in a vector, a gene such as *tob* is operably in expression ligated to, for instance, an inducible promoter, and the gene together with the promoter may be inserted into chromosome, so that expression can be controlled from the outside. In the construction of the vector, an incorporation-type vector such as YIp type vector is preferably used.

The transformation of the yeast with the above-mentioned vector can be carried out according to the conventional procedure. The yeast can be transformed by, for instance, lithium acetate method (Ito, H. et al., *J*. *Bacteriol.,* **153**, 163-168(1983)), electroporation method (Hashimoto, H. et al., *Appl. Microbiol. Biotechnol.,* **21**, 336-339(1985)), protoplast method (Hinnen, A. et al., *Proc. Natl. Acad. Sci. USA,* **75**, 1929-1933(1978)), and the like. After the procedures for the transformation, the transformed yeast is preferably screened depending upon the nature of a selectable marker gene by a conventional method. The resulting yeast comprises a gene encoding a heterogeneous protein (or a fragment thereof), and whether or not yeast comprises the gene can be confirmed by extracting a DNA from the yeast, and confirming with a restriction enzyme, or PCR method or a known nucleotide sequencing method. As the yeast of the present invention, an yeast comprising a gene encoding a heterogeneous protein (or a fragment thereof) operably in expression ligated to a promoter is preferable from the viewpoint of exhibiting the desired effects of the present invention.

In addition, the yeast deficient in aspiration ability can be easily obtained by a known method. The yeast can be grown under anaerobic conditions or aerobic conditions. The yeast acquires ATP necessary for the growth by glycolysis in cytoplasm in the former case, and by oxidative phosphorylation in the mitochondrion in the latter case. Therefore, by deleting a part or all of mitochondrial DNA, an aspiration ability-deficient strain (ρ⁻) is obtained (*Kobo no Kaibo (Anatomy of Yeast),* edited by Naohiko Yanagishima, Yasuharu Oshima, Masako Osumi, Kodansha Scientific, p137-147, 1981). An aspiration ability-deficient strain in which all of mitochondrial DNA are deleted is expressed by ρ⁰ in some cases. In the present specification, ρ⁻ and ρ⁰ are collectively represented as ρ⁻.

A concrete method of obtaining an aspiration ability-deficient strain (ρ⁻) from an yeast having aspiration ability (parent strain ρ⁺) is described, for instance, in *Laboratory Course Manual for Methods in Yeast Genetics,* Year 2000 Edition, authored by Burke, D., Dawson, D., Steams, T., Cold Spring Harbor Laboratory Press (2000). In other words, the aspiration ability-deficient strain can be easily obtained by culturing a parent strain in a medium containing ethidium bromide, and thereafter separating a strain which can be grown in a medium containing glucose as a carbon source, but cannot be grown in a medium containing glycerol as a carbon source. Alternatively, the aspiration ability-deficient strain can be obtained from a single colony of the parent strain, with, however, a lower frequency.

The yeast which can be used in the preparation of the yeast of the present invention having the desired properties as mentioned above includes a group of microorganisms described as yeasts in, for instance, *Bergey's Manual of Systematic Bacteriology, Second Edition,* authored by Boone, Castenholz, published by Lippincott Williams and Wilkins (2000). The kind of the yeast is not particularly limited. The yeast includes yeasts belonging to *Saccharomyces, Schizosaccharomyces, Kluyveromyces, Pichia* and the like. In the present invention, it is preferable to use the yeast belonging to *Saccharomyces* or *Schizosaccharomyces,* and it is more preferable to use, for instance, *Saccharomyces cerevisiae, Schizosaccharomyces pombe* or the like. Incidentally, the yeast belonging to *Kluyveromyces* includes, for instance, *Kluyveromyces lactis,* and the yeast belonging to *Pichia* includes, for instance, *Pichia pastoris.*

Whether or not the resulting yeast is an yeast having desired properties can be evaluated by confirming stability of the growing state of the yeast in a non-expression state of a heterogeneous protein and/or a change in the growing state of the yeast in an expression state of a heterogeneous protein and/or expression of the protein. Concretely, for instance, the evaluation can be made according to a method described in Example A-1 set forth below. Incidentally, since on and off of the expression state and the non-expression state cannot be controlled when a non-inducible promoter is used, in evaluating the properties of the yeast using the promoter, as the yeast in the non-expression state, there is used an yeast obtained by the same procedures as the yeast of the present invention except for the use of a vector without containing a gene encoding a heterogeneous protein. On the other hand, when an inducible promoter is used, the yeast can be also used as the yeast in a non-expression state.

The resulting yeast can be properly stored for some time period until use. As storage conditions, preferable are the conditions of inoculating cells into a liquid medium, culturing the cells until a stationary phase is reached, harvesting the cells from the culture medium, and suspending precipitates in a 10% glycerol solution, and storing the suspension in a freezer at -80°C.

The yeast of the present invention expresses a heterogeneous protein, and as a result, the yeast has the properties that a change in the growing state is caused. Although the details of the mechanism thereof are yet unknown, it is considered that the heterogeneous protein acts on a regulatory mechanism relating to proliferation and/or differentiation of an yeast for causing the change.

The yeast of the present invention is suitably used in the method of screening a physiologically active substance of the present invention. Since the yeast has the above-mentioned properties, various applications are expected in studies of an acting factor involved in proliferation or differentiation of cells, and action mechanisms thereof. Therefore, as one embodiment of the present invention, there are provided a reagent for screening a physiologically active substance, a reagent for studying an acting factor involved in proliferation or differentiation of cells, and action mechanisms thereof, each comprising the yeast of the present invention. The reagent may comprise the yeast of the present invention itself, or the reagent may optionally further contain other components as desired. For instance, it is possible to provide the reagent in a form in the above-mentioned storage state.

The method of screening a physiologically active substance of the present invention uses the yeast of the present invention as described above. Concretely, the method comprises the steps of:
(1) contacting a transformed yeast with a test sample, wherein the transformed yeast is capable of expressing a heterogeneous protein, and shows a change in a growing state in an expression state of the protein as compared to that in a non-expression state of the protein;
(2) culturing the yeast under conditions that the protein is capable of being expressed; and
(3) measuring the growing state of the yeast.
And the physiologically active substance is judged to be present in the test sample in a case where the growth of the yeast is lowered or improved in the presence of the test sample as compared to that in the absence of the test sample as a control.

In the yeast of the present invention, as mentioned above, it is considered that an expressed heterogeneous protein acts on a regulatory mechanism relating to proliferation and/or differentiation to cause a change in the growing state (for instance, lowering or improvement in growth). Therefore, in a case where an yeast expressing a heterogeneous protein is cultured in the presence of a test sample, for instance, in the case where the yeast is an yeast of which growth is lowered by expression of a heterogeneous protein, when the growth of the yeast is improved as compared to that in the absence of a test sample, or where the yeast is an yeast of which growth is improved by expression of a heterogeneous protein, it is considered that a component in a test sample is bound to, for instance, a heterogeneous protein or the like, to give some sort of influences on the action of the protein or the like, when the growth of an yeast is lowered as compared to that in absence of a test sample. For instance, in the case where a heterogeneous protein expressed in the yeast is Tob, when the growth of the yeast is improved in the presence of a test sample as compared to that in the absence of the test sample, there is a possibility that a component in the test sample inhibits the function of Tob. In addition, for instance, in the case where heterogeneous proteins expressed in the yeast are both Tob and Caf1, since it is known that Caf1 exhibits its effects by the interaction with Tob, when the growth of the yeast is improved in the presence of a test sample as compared to that in the absence of the test sample, there is a possibility that a component in the test sample can bind to Caf1, and exhibits a Tob-like action.

In the yeast of the present invention used in the present invention, as mentioned above, an inducible promoter or a non-inducible promoter can be used. Hereinbelow, for the sake of simplification in the explanation, the case where yeast obtained by using an inducible promoter capable of performing on and off of expression and easily controlling expression of a heterogeneous protein is used will be described.

The test sample is not particularly limited. The test sample includes, for instance, a synthetic compound which is chemically synthesized, an extract obtained from a tissue of a plant or an animal, and a compound purified therefrom, a culture medium of an microorganism, or plant or animal cells, a compound extracted and purified therefrom and the like. The sample may be properly diluted with an optional buffer or the like.

The method of contacting an yeast with a test sample in the step (1) is not particularly limited. For instance, while the step (2) comprises carrying out the culture (main culture) of the yeast under conditions that a heterogeneous protein is expressed, in other words, under conditions that a change in the growing state is caused, it is, for instance, preferable that the contact of an yeast with a test sample is carried out by adding a test sample simultaneously with the addition of a seed culture medium to a culture medium for the main culture in an embodiment (Embodiment 1) where the yeast is pre-cultured before the main culture under conditions that a heterogeneous protein or the like is not expressed, in other words, under conditions that a change in the growing state is not caused, to give a seed culture medium, and the seed culture medium is added to a main culture medium under conditions that a change in the growing state is caused to carry out main culture. In addition, it is, for instance, preferable that the contact of an yeast with a test sample is carried out by adding a test sample simultaneously with the addition of a component to a culture medium for main culture in an embodiment (Embodiment 2) where the yeast is cultured for several minutes to several hours or so in the main culture, and thereafter given components are added to a medium depending upon a promoter for controlling expression of a heterogeneous protein or the like, thereby making conditions that a change in the growing state is caused.

The pre-culture is carried out, for instance, by inoculating a properly stored yeast into a known medium suitable for culturing an yeast. The culture is carried out under usual culture conditions for an yeast, for instance, at 24° to 37°C for 2 to 3 days under aerobic or anaerobic conditions. In addition, the culture may be carried out, for instance, in a flask, a test tube or the like.

The main culture can be carried out under the similar conditions to those of the pre-culture. However, a medium under conditions that a change of the growing state of yeast is caused is used in the case of Embodiment 1. For instance, when a gene encoding a heterogeneous protein is ligated under control of *GAL1* promoter, a medium with addition of galactose to the medium used in the pre-culture may be used. Alternatively, the main culture may be carried out in wells of a microtiter plate, for the reasons such as numerous test samples can be tested simultaneously therein.

The growing state of yeast in a step (3) is determined by measuring any parameters which serve as an index of an extent of proliferation of cells in a culture process of an yeast. It is preferable that the growing state of yeast is determined concretely by, for instance, monitoring a change in turbidity of an yeast culture medium, a morphological change of the yeast, a change in wet-weight of the yeast, a change in dry-weight of the yeast, a change in endogenous enzyme activity of the yeast or a change in amount of an endogenous protein of the yeast, without being limited to these methods. The change in turbidity of an yeast culture medium can be monitored by measuring turbidity of a culture medium during the main culture with, for instance, a spectrophotometer over time. Similarly, the morphological change of the yeast can be monitored by, for instance, observing the morphology of the yeast with an optical microscope over time. The change in wet-weight of the yeast can be monitored by collecting a culture medium over time and harvesting cells (for instance, by centrifugation at 3,000 rpm for 10 minutes), and determining the weight thereof, and the change in dry-weight of the yeast can be monitored by drying harvested yeast (for instance, by keeping at 105°C for 4 hours), and then determining the weight thereof, respectively. The change in endogenous enzyme activity of the yeast can be monitored, for instance, by assaying activity of invertase or hexokinase over time by a conventional method. In addition, the change in amount of an endogenous protein of the yeast is preferably monitored by a quantitative change of the protein of which expression level does not change depending upon the growth stage. For instance, the change can be monitored by determining an amount of actin or URA3 over time by Western blotting with an antibody specific to the protein. Incidentally, since the change in the growing state of the yeast of the present invention may be deflected during the course of the culture due to various causes in some cases, the monitoring is carried out for preferably from 10 to 300 hours, more preferably from 80 to 200 hours.

On the other hand, as a control the same procedures are carried out except that the yeast and the test sample are not contacted with each other in the step (1), and the growing state of yeast is measured in the same manner as described above.

Next, whether or not lowering or improvement in the growth of the yeast is found in the presence of the test sample as compared to that in the absence of the test sample as a control is evaluated based on the obtained results. It is preferable that the evaluation is made by plotting the measurement results obtained of the growing state of the yeast against time to obtain a proliferation curve of the yeast, and comparing the resulting proliferation curve of the yeast contacted with the test sample with a proliferation curve of the control. Concretely, proliferation curves of the yeast in the presence and in the absence of a test sample are obtained, and each of generation time is obtained to be compared. When the generation time of the yeast is longer in the presence of the test sample as compared to that in the absence of the test sample, it is evaluated that lowering in the growth of the yeast is found in the presence of the test sample as compared to that in the absence of the test sample as a control. On the other hand, when the generation time of the yeast is shorter in the presence of the test sample as compared to that in the absence of the test sample, it is evaluated that improvement in the growth of the yeast is found in the presence of the teat sample as compared to that in the absence of the test sample as a control. Alternatively, the proliferation rate may be obtained in place of the generation time. In this case, when the proliferation rate is delayed, it is evaluated that lowering in the growth of the yeast is found in the presence of the test sample as compared to that in the absence of the test sample as a control. In addition, when the proliferation rate is increased, it is evaluated that improvement in the growth of the yeast is found in the presence of the test sample as compared to that in the absence of the test sample as a control. Incidentally, when the growing state of yeast is measured by monitoring a morphological change of the yeast, the number of cells in which the morphological change takes place is counted with a hematometer or the like, and a proliferation curve is obtained by plotting the number of cells against culture time of the yeast.

From the results mentioned above, when lowering or improvement in the growth of the yeast is found in the presence of the test sample, it is judged that a physiologically active substance is present in the test sample. Since the physiologically active substance has an activity capable of causing some sort of influences on a change in the growing state of the yeast of the present invention accompanied by expression of a heterogeneous protein, for instance, capable of canceling the change, the substance can be isolated using the activity as an index according to a known purification method by further combining, for instance, centrifugation, filtration, liquid-liquid distribution, adsorption/elution, chromatography, recrystallization and the like as desired.

In addition, the present invention provides a physiological active substance obtainable by the above-mentioned method of screening. The physiologically active substance has a physiological activity at least associated with a function of a heterogeneous protein used in the screening process. For instance, when a heterogeneous protein is a signal protein, since it is considered that the physiologically active substance is capable of acting on control mechanism of cell cycle, there are expected, for instance, use in studies on elucidation of control mechanism of cell cycle, or use in treatment or prevention for diseases and the like in which abnormality of control mechanism of cell cycle is at least one of causes for incidence or pathological progression. For instance, there is a possibility that a substance for inhibiting a function of Tob is a therapeutic agent for bone disease such as osteoporosis and fracture, and there is a possibility that a substance interacting with Caf1 to exhibit the similar action to that of Tob is an anti-cancer agent.

According to the method of screening, for instance, a tyrosine kinase inhibitor can be obtained as a physiologically active substance, such the substance has a function of inhibiting phosphorylation of a tyrosine residue of a protein, and the inhibitor can be used, for instance, as a therapeutic for diseases in which excessive intracellular signaling associated with tyrosine kinase is a causative for exacerbation of pathology, more concretely, as a cancerocidal agent or the like.

When the physiologically active substance of the present invention is formed into a pharmaceutical preparation, the pharmaceutical preparation can be prepared as a solid, semi-solid or liquid preparation for oral or parenteral administration, for instance, using a known organic or inorganic carrier, an excipient and other additives suitable for oral or parenteral administration according to a conventional method. The dose of the physiologically active substance may be properly adjusted depending upon the properties of each substance so that the desired effects can be obtained. In addition, the content of the physiologically active substance in the pharmaceutical preparation is not particularly limited as long as a given amount of the physiologically active substance can be administered depending upon its method of use.

Further, as one embodiment of the present invention, a kit for screening a physiologically active substance comprising the yeast of the present invention is provided. The kit can be suitably used in the method of screening a physiologically active substance of the present invention. The kit may comprise a given medium, various reagents used for measuring the growing state of the yeast (for instance, an reagent for quantifying protein, a plasmid vector for transformation, a host yeast, a specific antibody for Tob or the like, reagent for quantifying enzyme activity and the like), and an instruction manual for carrying out the method of screening a physiologically active substance of the present invention.

### EXAMPLES

The present invention will be described more specifically by means of the following Examples, without intending to limit the present invention to the Examples.

### (A) Examples in Yeasts Having Aspiration Ability

Strains, media, reagents, and general experimental methods used in the following examples are summarized as follows:

### 1. Strains

Strains used in the present experiment are shown in Table 1.

**Table 1**

| Microorganism | Strain | Genotypes |
|---|---|---|
| *E. coli* | JM109 | *hsd*R17(rK⁻, mK⁺), *rec*A1, *end*A1, *Δ*(*lac-pro*AB), *gyr*A96, *thi-*1, *rel*A1*, sup*E44, F'[*tra*D36,*pro*AB, *lac*I^{*q*}*, lac*ZΔM15], e14^{*-*}(*Mcr*A^{*-*}) |
| *S. cerevisiae* | YPH500 | *MATα, ura3-52, lys2-801*^{amber}, *ade2-101*^{ochre}, *trp1-Δ63, his3-D200, leu2-Δ1* |
| *S. cerevisiae* | YNN27 | *MATα, trpl, ura3* |
| *S. cerevisiae* | 20B-12 | *MATα, trp1, pep4* |
| *S. cerevisiae* | INVSc1 | *MATα, his3-Δ1, leu2, trp1-289, ura3-52* |

### 2. Media

All of the media were autoclaved and used. As to the compositions of each of the media, reference is made to *Laboratory Course Manual for Methods in Yeast Genetics, 2000 Edition,* authored by Burke, D., Dawson, D., and Stearns, T., Cold Spring Harbor Laboratory Press (2000) for the media for yeasts, and reference is made to Sambrook et al., *Molecular Cloning A Laboratory Manual: A Laboratory Manual 2nd ed.,* Cold Spring Harbor Laboratory Press (1989) for the media for *E*. *coli.*
(1) *E. coli* cells were cultured using the following media.
   - LB liquid medium
   - LB solid medium
   - LB + Amp(ampicillin) medium (Amp concentration: 100 µg/ml)
(2) *E. coli* cells were transformed using the following media.
   - SOB medium
   - SOC medium
(3) Yeast host cells were cultured using the following media.
   - YPD liquid medium
   - YPD solid medium
(4) Transformant of an yeast was cultured using the following media.
   - CSM liquid medium
   - CSM solid medium
   - CSM-TRP medium
   - CSM-URA medium
   - CSM-TRP-URA medium

### 3. Reagents

As to the compositions of each of the reagents, reference is made to *Laboratory Course Manual for Methods in Yeast Genetics, 2000 Edition,* authored by Burke, D., Dawson, D., and Steams, T., Cold Spring Harbor Laboratory Press (2000) for yeasts, and reference is made to Sambrook et al., *Molecular Cloning A Laboratory Manual: A Laboratory Manual 2nd ed.,* Cold Spring Harbor Laboratory Press (1989) *for E. coli.*
- TB (Transformation Buffer)
- 3 M Sodium acetate (NaOAc) solution (pH 4.8)
- Glucose buffer
- 1 % SDS-0.2N NaOH (solution II for plasmid extraction)
- TE
- 10 × TBE
- Acrylamide: bis(30:0.8)
- 1.5 M Tris-HCl buffer (pH 8.8)
- 0.5M Tris-HCl buffer (pH 6.8)
- Sample buffer
- Electro-blotting buffer
- PBS
- TPBS
- 0.2 M Lithium acetate (LiOAc) solution
- 70% Polyethylene glycol (PEG)

### 4. Experimental Methods

### (1) Transformation of Escherichia coli JM109

One-hundred microliters of competent cells of JM109 stored at -80°C were thawed on ice, and 5 µl of plasmid DNA was added thereto, and the mixture was allowed to stand on ice for 30 minutes. After the mixture was subjected to heat shock at 42°C for 30 seconds, the resulting mixture was allowed to stand for 2 minutes on ice, and 0.9 mL of a SOC medium was added thereto. The components were thoroughly mixed, and the cells were cultured at 37°C for 45 minutes. The resulting culture was plated on an LB + Amp solid medium having an Amp concentration of 100 µg/ml, and the cells were cultured at 37°C overnight.

### (2) Method of Preparing Medium Scale of Plasmid DNA (Medium-Scale Extraction)

Cells previously cultured in 30 ml of the LB + Amp liquid medium were transferred to a centrifugation tube, and the cells were harvested by centrifuging the culture at room temperature for 5 minutes at 8000 rpm. To the precipitates was added 3 ml of a lysozyme solution (5 mg lysozyme/1 ml glucose buffer) to gently lyse the precipitates, and the mixture was allowed to stand on ice for 5 minutes. Thereto was added 4 ml of an NaOH-SDS solution (0.2 N NaOH - 1% SDS) while gently mixing, and the mixture was allowed to stand on ice for 5 minutes. Thereto was added 3 ml of a 3 M NaOAc solution (pH 4.8) while gently mixing, and the mixture allowed to stand on ice for 10 minutes, and centrifuged at 4°C for 15 minutes at 15,000 rpm. The supernatant was transferred to a fresh tube, and 6 ml of isopropanol was added thereto. The mixture was allowed to stand at -20°C for 20 minutes, and centrifuged at 4°C for 15 minutes at 15,000 rpm. The precipitates were washed with 2 ml of 70% ethanol, dissolved in 300 µl of TE, and transferred to an Eppendorf (registered trademark) tube. The centrifugation tube was similarly washed with 300 µl of TE, and the TE was transferred to the Eppendorf (registered trademark) tube (total of 600 µl). Thereto was added 6 µl of 10 mg/ml RNase, and the mixture was incubated at 37°C for 20 minutes. The mixture was extracted three times with phenol, extracted three times with chloroform, and extracted three times with ether. To the resulting extract were added 120 µl of a 5 M NaCl solution and 240 µl of 30% PEG #6000 while mixing, and the mixture was allowed to stand at -20°C for 40 minutes, and allowed to stand at 4°C for 30 minutes. After the mixture was centrifuged at 4°C for 10 minutes at 14,000 rpm, the precipitates were harvested with complete removal of water from the tube, and dissolved in 80 µl of sterile water. Thereto were added 4.8 µl of a 5 M NaCl solution and 192 µl of ethanol while mixing, and the mixture was allowed to stand at -20°C for 30 minutes. After the mixture was centrifuged at 4°C for 15 minutes at 14,000 rpm, 200 µl of 70% ethanol was added to the precipitates to gently wash the precipitates. After the mixture was centrifuged at 4°C for 15 minutes at 14,000 rpm, the precipitates were dried with a suction pump, and dissolved in 100 µl of TE. Thereafter, its concentration was determined by an OD₂₆₀ value and electrophoresis.

### (3) PCR Method

PCR was performed for 25 cycles under the conditions that one cycle comprises 98°C for 30 seconds, 60°C for 60 seconds and 72°C for 90 seconds. The reaction was performed by adding 5 µl each of 50 µM primers, 10 µl of NEB × 10 buffer, 10 µl of a 4 mM 4dNTP mix, 0.5 µl of NEB Ventpol (2U/µl) and 59.5 µl of sterile water to 10 µl of a 5 ng/µl sample DNA to adjust its volume to a total of 100 µl, and adding 1 µl of 10 mg/ml BSA and 60 µl of a mineral oil thereto.

### (4) Isolation of DNA by Cut-out from Agarose Gel

The amount 5 µg of plasmid DNA prepared by the medium-scale extraction was treated with a restriction enzyme in a 100 µl system, and thereafter 20 µl of Loading Dye was added thereto so as to make up a volume of 120 µl. Three combs having a width of 6 mm of an electrophoretic apparatus "MUPID (registered trade mark)" (manufactured by ADVANCE Co., Ltd.) were wound with a cellophane tape so as to gather those three combs into one. A 120 µl of a sample was applied to a gel, and electrophoresed. After the termination of electrophoresis, a desired band was cut out with a cutter knife while irradiating a gel with a 365 nm long-wavelength DNA cut-out lamp. The collected gel was placed into a dialysis tube containing 250 µl of 0.5 × TBE, and the dialysis tube was set in Mupid, and electrophoresis was carried out for 30 minutes in a usual direction, and thereafter for 30 seconds in a reverse direction. The 0.5 × TBE was collected, and extracted twice with phenol, and extracted three times with ether.

### (5) Small-Scale Process for Preparing Plasmid DNA (Mini-prep)

A colony of transformed *Escherichia coli* was inoculated into a 1.2 ml LB + Amp liquid medium contained in an Eppendorf (registered trademark) tube, and cultured overnight at 37°C. After the culture medium was centrifuged at room temperature for 1 minute at 12,000 rpm, 100 µl of a glucose buffer was added thereto, and the mixture was vortexed to completely suspend the precipitates. To the suspension was added 150 µl of an NaOH-SDS solution (0.2 N NaOH-1% SDS), the tube was shaken vertically to gently mix them, and thereafter the mixture was allowed to stand on ice for 5 minutes. 150 µl of a 3 M potassium acetate buffer was added thereto while mixing, and 500 µl of phenol was added thereto while mixing. Thereafter, the mixture was centrifuged at 4°C for 5 minutes at 12,000 rpm. The upper layer (about 500 µl being collected) was transferred to another tube, an equivolume (500 µl) of chloroform was added thereto, and the mixture was centrifuged at 4°C for 5 minutes at 12,000 rpm. The upper layer (about 400 µl being collected) was transferred to another tube, a twice the volume (800 µl) of ethanol was added thereto while thoroughly mixing, and the mixture was allowed to stand at -20°C for 30 minutes. Thereafter, the mixture was centrifuged at 4°C for 10 minutes at 14,000 rpm, and 200 µl of 70% ethanol was added to the precipitates while gently mixing. The mixture was centrifuged at 4°C for 5 minutes at 14,000 rpm, and the precipitates were dried with a suction pump, and then dissolved in 20 µl of RNase-containing TE (10 mg/ml RNase 1 µl-TE 20 µl), and incubated at 37°C for 30 minutes. Thereafter, the resulting plasmid DNA was treated with a restriction enzyme, and confirmed by electrophoresis.

### (6) Transformation of Yeast (Lithium Acetate Method)

An yeast was inoculated into 5 ml of an YPD liquid medium, and the mixture was pre-cultured overnight at 30°C. One milliliter of the culture medium was inoculated into 20 ml of an YPD liquid medium, and the cells were cultured at 30°C for 4 to 6 hours until a KU (Klett unit) value was adjusted to around 60. The OD₆₀₀ value of the culture medium was determined, and the amount of the culture medium was calculated from a found value so that the number of cells is 2 × 10⁸ cells. The calculated culture medium was weighed, and transferred to a 50 ml centrifugation tube. After the culture medium was centrifuged at room temperature for 5 minutes at 3,000 rpm, the precipitates were suspended in 10 ml of TE, and the suspension was similarly centrifuged at room temperature for 5 minutes at 3,000 rpm. The precipitates were suspended in 1 ml of TE, and 1 ml of a 0.2 M lithium acetate solution was added to the suspension. The mixture was cultured at 30 °C for 1 hour (termination of the preparation of competent cells). Two-hundred microliters each of the prepared competent cells were dispensed into a 1.5 ml tube, 5 µg each of the DNA was added thereto, and the mixture was allowed to stand on ice for 30 minutes. Thereto was added 200 µl of 70% PEG 4000 while thoroughly mixing. The mixture was incubated at 30°C for 1 hour, and then subjected to heat shock at 42°C for 5 minutes. Thereafter, the mixture was immediately centrifuged at 4°C for 5 minutes at 12,000 rpm, and the precipitates were suspended in 1 ml of sterile water chilled on ice in advance. The suspension was centrifuged at 4°C for 5 minutes at 12,000 rpm. After the precipitates were suspended in 100 µl of chilled sterile water, the suspension was plated on a CSM-TRP solid medium, and the cells were cultured at 30°C for 3 to 5 days. A KU value was determined with a Klett Summerson photoelectric photometer (manufactured by Klett Manufacturing).

### (7) SDS-PAGE-Western Blotting

### ① Preparation of Protein (Alkali-TCA-SDS Method)

A 5 ml culture medium was transferred to an Eppendorf (registered trade mark) tube, and the cells were harvested by centrifuging the culture medium at 14,000 rpm for 5 minutes. The procedures were repeated two or three times. The precipitates were suspended in 1.25 ml of sterile water, 160 µl of Me-NaOH was added thereto, and the mixture was allowed to stand on ice for 10 minutes. After the mixture was centrifuged at 14,000 rpm for 20 minutes, the supernatant was completely removed with a pipetteman. To the precipitates was added 1.5 ml of chilled acetone, and the cells were completely suspended by vortexing and ultrasonication treatment. After the suspension was allowed to stand on ice for 10 minutes, the precipitates were harvested by centrifuging the suspension at 14,000 rpm for 5 minutes. The cap of the Eppendorf tube was opened, and the precipitates were allowed to stand at room temperature to evaporate acetone (acetone washing). After the acetone washing was carried out again once more, the precipitates were dissolved in 150 µl of a 1% SDS/0.1M Tris-HCl buffer (pH 6.0), to confirm whether the pH was neutral or alkaline on a pH test paper. Thereafter, the precipitates were dissolved by ultrasonication treatment. After the solution was heated at 95°C for 3 minutes, the supernatant was collected by centrifuging the heated solution at 14,000 rpm for 5 minutes. The amount of protein was determined using a Lowry method.

### ② Lowry method

The present method is described in "Lowry, O. H., Rosebrough, N. J., Farr, A. L., and Randall, R. J. *(1951) J. Biol. Chem.* **193**, 265-275." First, a standard curve was prepared with BSA (bovine serum albumin). Each sample was prepared so that BSA was adjusted to concentration of 10, 20, 30, 40, 50, 70, 90, 110, or 130 µg/200 µl. To each sample was added 1 ml of a C solution, and the mixture was vortexed, and allowed to stand at room temperature for 10 minutes. A 0.1 ml Lowry reagent was added thereto, and the mixture was vortexed, and allowed to stand at room temperature for 30 minutes. The values for OD₇₅₀ and OD₅₀₀ were then determined, and a standard curve was prepared from the resulting values. Values of OD₇₅₀ and OD₅₀₀ of a desired sample were determined in the same manner as described above. The amount of protein was determined in reference to the standard curve.

### ③ Production, Preparation and Electrophoresis of Gel

This method was carried out by using an electrophoretic apparatus MODEL BE-230 (manufactured by BIO CRAFT). A 7% gel or a 10% gel was used as a separation gel, respectively. A glass flat plate, a glass plate, and a pad were cleanly wiped with 70% ethanol, and clipped together. A 10% APS (Ammonium Peroxodisulfate) was added to a separation gel, the gel was poured into the plate, and 1 ml of sterile water was poured thereon. After the gel was allowed to stand at room temperature for 30 minutes, sterile water was discarded, and the residual water was removed with a filter. A 10% APS was added to the concentration gel, and the gel was poured into a plate. A comb was inserted thereinto, and the gel was allowed to stand at room temperature for 30 minutes. The composition of a separation gel and that of a concentration gel are described in Laemmli, U.K.(1970) *Nature* **227**,680-685, 1970 or *Shin-Seikagaku Jikken Koza 1. Tanpakushitsu I Bunri, Seisei, Seishitsu (New Biochemistry Experimental Lecture 1. Protein I Separation, Purification, Nature),* edited by Japanese Biochemistry Association, Tokyo Kagaku Dojin Co., Ltd, 1997.

The comb of the prepared gel was removed, and the remaining gel in wells of the comb was scraped with a small dispensing spoon and washed with distilled water. A pad was removed, an upper layer of an electrophoresing layer was clipped together with both ends of the pad. An electrophoresing buffer was placed in a lower layer of an electrophoresing layer, and an upper layer was placed into the lower layer. A fresh electrophoresing buffer was placed in the upper layer, and a marker and a sample were applied. Thereafter, an electric cord was connected, and electrophoresis was carried out at an electric current of 30 mA. After the termination of electrophoresis, the clip was removed, and only the separation gel was collected, and transferred into a Tupperware.

### ④ Western Blotting

Six pieces of filters and a nitrocellulose membrane having the same size as the gel immersed in an Electro-blotting buffer were overlaid on an electrode plate for Western blotting in the order of three pieces of the filters, the gel, the nitrocellulose membrane, and three pieces of the filters, and an electric current was applied at 100 mA for 1 hour. After the termination of application of the electric current, the nitrocellulose membrane was transferred to a plastic case, shaken in TPBS for 1 hour (solution being exchanged every 20 minutes), and shaken in 5 ml of TPBS with addition of 5 µl of a primary antibody, for 1 hour. After shaking in TPBS for 30 minutes (solution being exchanged every 10 minutes), and the membrane was shaken for 1 hour in 5 ml of TPBS with addition of 5 µl of a secondary antibody. After shaking in PBS for 30 minutes (solution being exchanged every 10 minutes), the nitrocellulose membrane was placed in a solution prepared by dissolving 6 mg of 4-chloro-1-naphthol in 2 ml of methanol, with adding 10 ml of PBS and 5 µl of H₂O₂ thereto, and shaken with hands to emit light. After shaking in sterile water for 15 to 30 minutes, the membrane was stored by wrapping with a filter and an aluminum foil.

### Example A-1 Expression 1 of tob (htob) gene in yeast Saccharomyces cerevisiae

In this example, Tob protein was expressed in cells by introducing *tob* gene into an yeast *Saccharomyces cerevisiae* YPH500α strain, and the change in the growing state of the yeast caused thereby was observed. In other words, a plasmid for introducing *tob* gene was constructed, an yeast was transformed with the constructed plasmid, and the growing state thereof was observed.

As a vector, pESC-TRP was used for subcloning. In addition, as *tob* gene, pME18S-*tob* containing the gene (one in which *tob* gene was inserted into *Eco*RI site of pME18S) was amplified, and the resulting gene was used.

### (1) Preparation of pESC-TRP-tob

*Escherichia coli* JM109 was transformed with pME18S-*tob*, and plasmid DNA was prepared from the resulting transformant (medium-scale extraction). *Escherichia coli* was transformed with pESC-TRP, and plasmid DNA was prepared in the same manner (medium-scale extraction). PCR was performed with the prepared plasmid pME18S-*tob* as a template using
a forward primer:
   5'-cccggatcca tgcagcttga aatccaagta-3' (SEQ ID NO: 5), and
a reverse primer:
   5'-cccgtcgacg ttagccataa caggctggaa-3' (SEQ ID NO: 6)
to amplify *tob* gene. The resulting PCR product was treated with *Bam*HI and *Sal*I, and DNA was then collected by cut-out from the gel. As to pESC-TRP, after the treatment with *Bam*HI and *Sal*I, and DNA was collected by cut-out from the gel. The ligation was carried out with *tob* gene and a fragment of pESC-TRP collected by these experimental procedures, and *Escherichia coli* was transformed with the resulting product. After the resulting transformant was confirmed by a mini-prep, plasmid DNA was prepared (medium-scale extraction).

### (2) Expression of Tob Protein in YPH500α Strain and Growth Experiment

An YPH500α strain was transformed with the prepared plasmid according to the lithium acetate method. The resulting transformant was subcultured on a different CSM-TRP solid medium, and cultured at 30°C for 2 to 3 days, and thereby a tryptophan-requiring transformant was screened. A colony was inoculated from the plate into 5 ml of a CSM-TRP Glc liquid medium, and the colony was cultured at 30°C for 2 to 3 days while shaking. A 0.1 ml culture medium each was subcultured on two 5 ml of CSM-TRP Gal liquid media and one CSM-TRP Glc liquid medium, and cultured at 30°C for 4 to 7 days while shaking. The KU values of the CSM-TRP Gal liquid medium in which a Tob protein was expressed were determined every 2 hours during the culture, and the growing state of the yeast was observed thereby. Separately, the protein was collected from another culture medium of CSM-TRP Gal liquid medium, and expression of Tob protein was confirmed by Western blotting. The yeast inoculated into the CSM-TRP Glc liquid medium was cultured to an intermediate phase of a logarithmic growth phase, washed with 5 ml of a CSM-TRP Gal liquid medium, and suspended. The yeast was cultured at 30°C for several hours, and a protein was collected, and subjected to Western blotting.

### (3) Analysis of Growth Inhibition of YPH500α Strain by Expression of Tob Protein

A pESC vector, which is a protein expression vector for an yeast, has a GAL1 promoter, and expresses a desired gene only in the presence of galactose. In order to evaluate the change in the growing state of the YPH500α strain by expression of *tob* gene, the growth of the yeast was evaluated by first pre-culturing the yeast in a medium containing glucose, and thereafter subculturing the pre-culture in a medium containing galactose, thereby expressing *tob* gene.

As an experimental method, a transformant of an YPH500α strain by pESC-TRP or pESC-TRP-*tob* was inoculated into 5 ml of the CSM-TRP Glc liquid medium using a tip of a toothpick, cultured at 30°C for 2 to 3 days while shaking at 170 rpm, and cultured to full growth. Thereafter, 100 µl was taken from the culture medium, inoculated into 5 ml of a CSM-TRP Gal liquid medium, and cultured for 5 to 7 days under the same conditions. A KU value was determined every 2 hours, and the growing state of each yeast was evaluated.

### Expression of tob Gene in YPH500α Strain

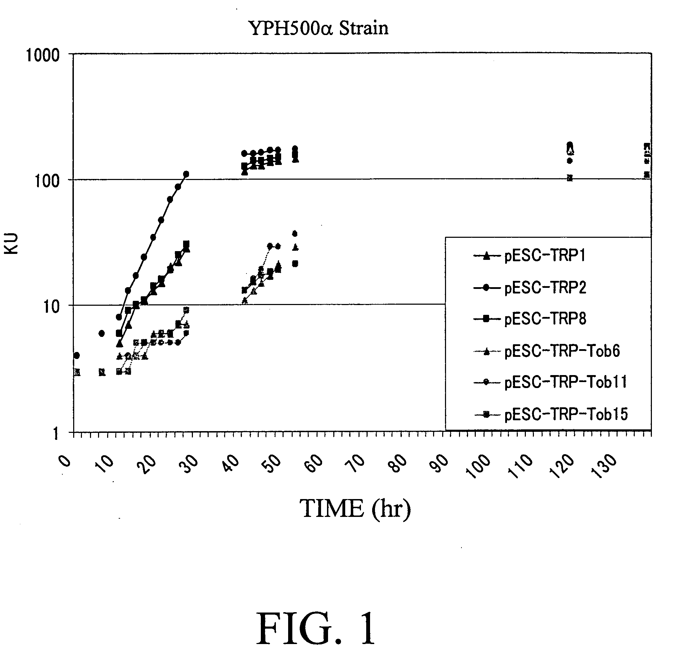

The KU values were plotted for the YPH500α strain against the culture time, and a proliferation curve was prepared. The results are shown in Figure 1.

In the present specification, in the figures, the name of each plasmid shows the results of an yeast transformed with the plasmid, and the number after the name of each plasmid shows a different transformed yeast obtained using the plasmid.

When *tob* gene-incorporated pESC-TRP-*tob* was introduced, the growth was clearly worsened, as compared to the control into which pESC-TRP consisting only of a vector was introduced. In other words, when a generation time and a proliferation rate are obtained, it can be determined that the growing state is inhibited for pESC-TRP-tob-6, 11 or 15 because the generation time becomes longer, and the proliferation rate becomes smaller in pESC-TRP-tob-6, 11 or 15, as compared to pESC-TRP-1, 2 or 8. It can be seen from the results that expression of Tob protein in an YPH500α strain inhibits the growth of an yeast. In this respect, *tob* gene suppresses growth for 2 or 3 days from the beginning of culture, and it could be also seen that at a stage where 120 hours have passed, the growth catches up with the growth for the case consisting only of a vector, which is the control. It is thought that since Tob does not make the yeast die but suppresses the growth (growth rate is delayed), the yeast reaches a stationary phase by long-term culture, and catches up with the growth of the control yeast. In addition, since the used plasmid is a multicopy plasmid, its stability is unknown, so that it is also thought that a plasmid is dropped off at the stage of culture, thereby decreasing an expression level of the Tob protein. In addition, it is thought that an yeast resistant to the Tob protein appears due to occurrence of spontaneous mutation at the stage of culture, and only the yeast survives, and continues to proliferate, and finally proliferation catches up to the same level as that of the control. Since Tob is a protein which is very easily degradable, it is also thought that the growth is recovered by degradation of Tob.

The test was performed additionally twice using the transformed yeast mentioned above and in the same manner using another transformed yeast, and a similar growth inhibition was observed as described above.

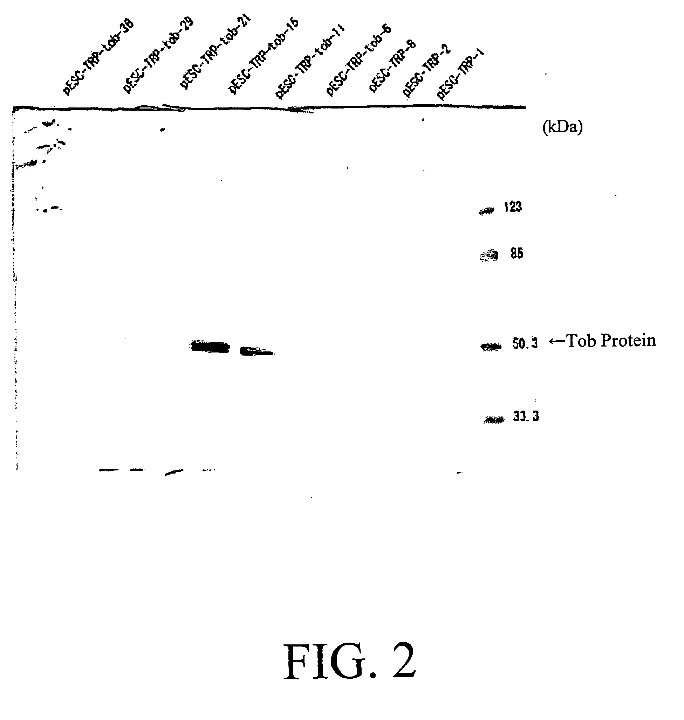

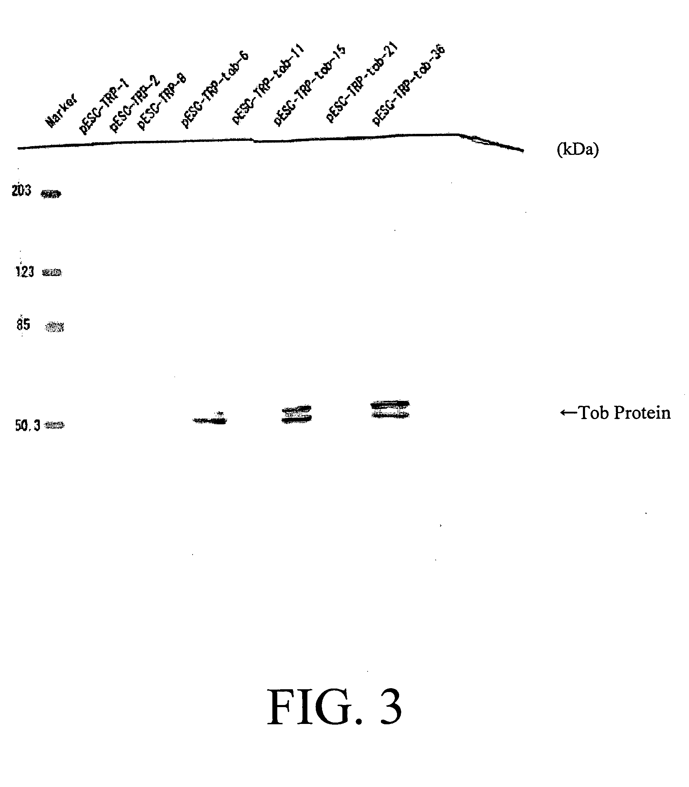

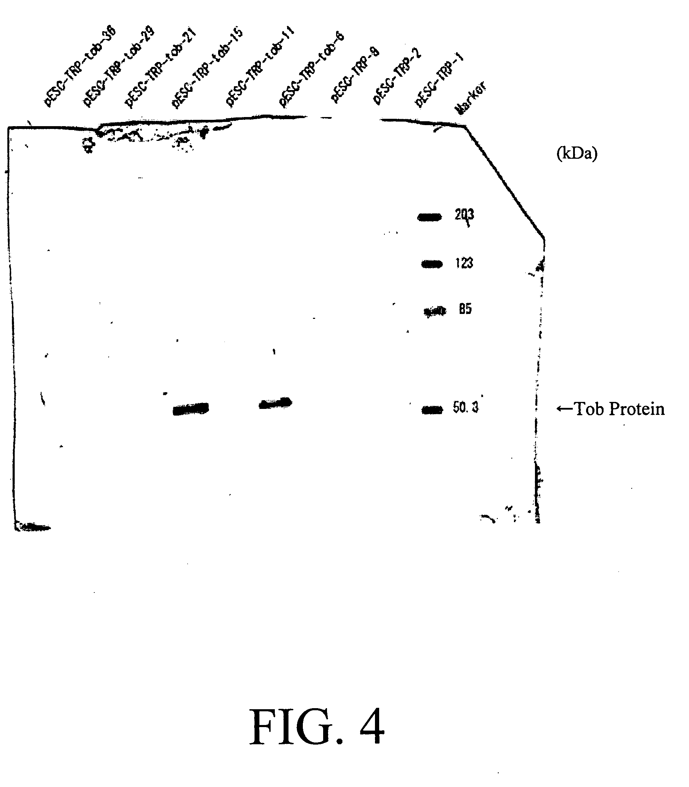

In addition, the culture was stopped at a point where the yeast was cultured for 9 hours, 78 hours or 181 hours in a different experiment using the same transformed yeast as described above, and the culture medium was subjected to Western blotting. A 0.1 ml pre-cultured CSM-TRP Glc liquid medium each was inoculated into another one CSM-TRP Glc liquid medium, and with two CSM-Gal liquid media. The yeast was cultured in the CSM-TRP Glc liquid medium until a point immediately before reaching a stationary phase, and the cells were harvested, washed with a CSM-TRP Gal liquid medium, suspended in a CSM-TRP Gal liquid medium, and cultured for 9 hours. The culture medium was subjected to Western blotting. One of the CSM-TRP Gal liquid medium was used for preparing a proliferation curve, and the culture for another medium was stopped at a point where the yeast was cultured for 78 hours (at a time point where there is a difference in growth). The culture medium was subjected to Western blotting. The culture was stopped for the culture medium previously cultured for preparing a proliferation curve at a point where the yeast was cultured for 181 hours. The culture medium was subjected to Western blotting. The results at a point where the yeast was cultured for 9 hours are shown in Figure 2, the results at a point where the yeast was cultured for 78 hours are shown in Figure 3, and the results at a point where the yeast was cultured for 181 hours are shown in Figure 4. In each case, although the Tob protein was not captured in some cases depending upon the experimental conditions, the Tob protein was captured under any of experimental conditions for every yeast. Therefore, the Tob protein was substantially captured in all the cases. In other words, there were obtained the results that the Tob protein was captured in all the cases where Tob was expressed in a medium containing galactose, and a protein was collected at a point where there is a difference in growth. A Tob antibody (Figures 2 and 4) and a myc antibody (Figure 3) were used for detecting the Tob protein. The myc antibody is an antibody specific for a myc tag added to C-terminal of Tob protein.

### Example A-2 Expression 2 of tob Gene in Yeast S. cerevisiae

The transformants of *S*. *cerevisiae* YNN27 strain and 20B-12 strain were respectively obtained, and the growing states of the yeasts were evaluated in the same manner as in Example A-1.

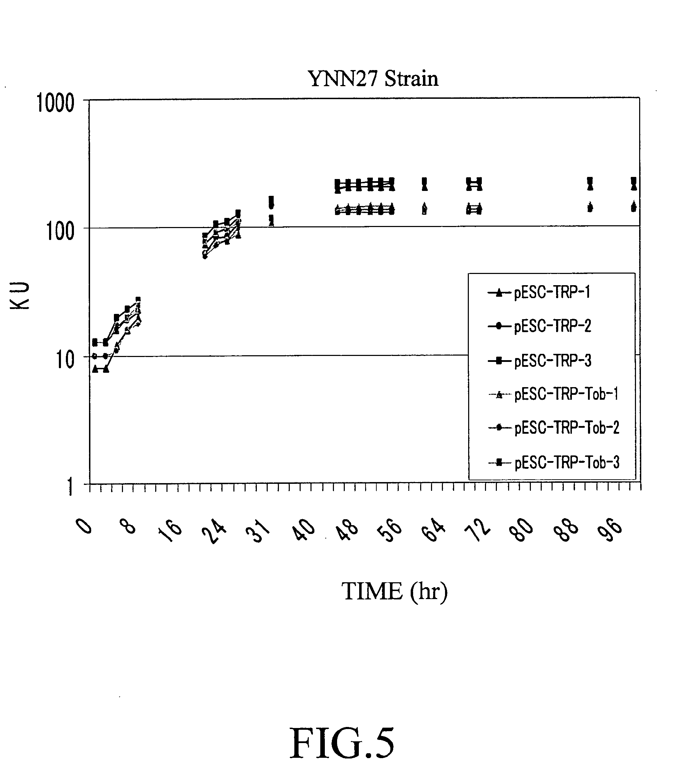

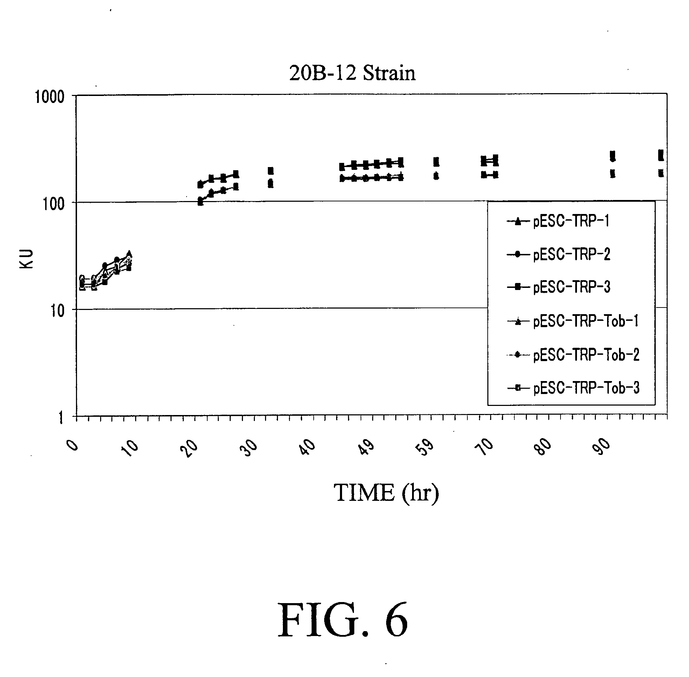

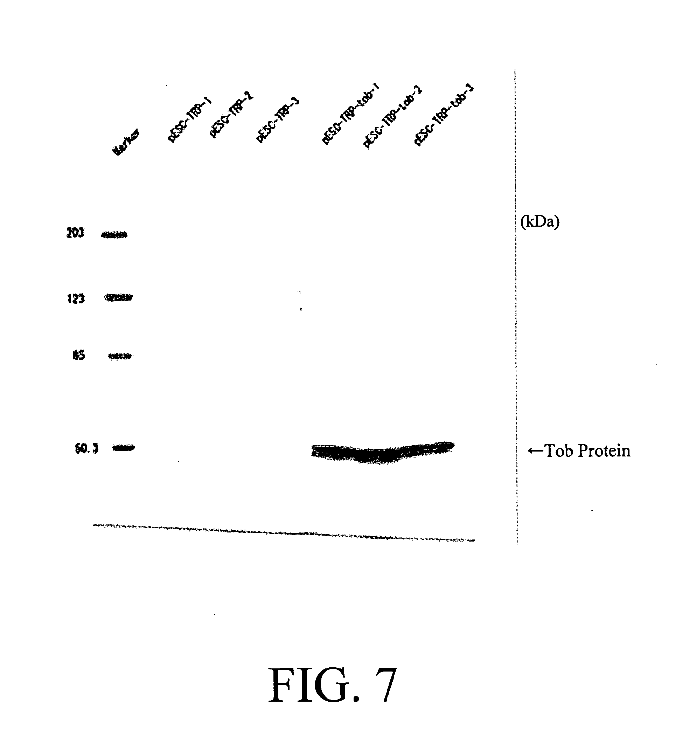

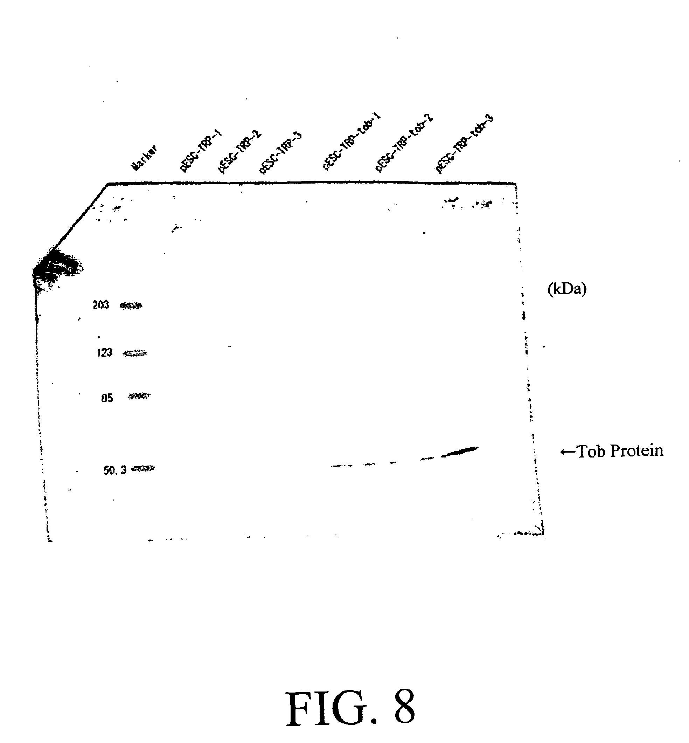

A proliferation curve was prepared for each of the transformants. The results are shown in Figures 5 and 6. In these strains, although there was little difference in growth for some time after the beginning of culture, a slight difference was found after about 48 hours for the YNN27 strain, and after 24 hours for the 20B-12 strain. Moreover, in this case, even when the culture was continued, the difference was not diminished, and the difference was held persistently. In this case, a 0.1 ml pre-cultured CSM-TRP-Glc liquid medium was inoculated into another CSM-TRP Glc liquid medium and a CSM-Gal liquid medium, respectively, and the CSM-Gal liquid medium was used for preparing a proliferation curve. The CSM-TRP Glc liquid medium was cultured to a point just before full growth, and the cells were harvested, washed with a CSM-Gal liquid medium, suspended in a CSM-Gal liquid medium, and cultured for 5 hours. The culture was subjected to Western blotting. The protein was not quantified by a Lowry method, but a proper amount of the protein was applied. The results for the YNN27 strain are shown in Figure 7, and the results for the 20B-12 strain are shown in Figure 8 (the myc antibody being used in the detection of the protein). As is seen from the results, expression of the protein was clearly seen in all the samples into which *tob* gene was introduced.

### Example A-3 Confirmation of Presence of Plasmid DNA in Transformed Yeast

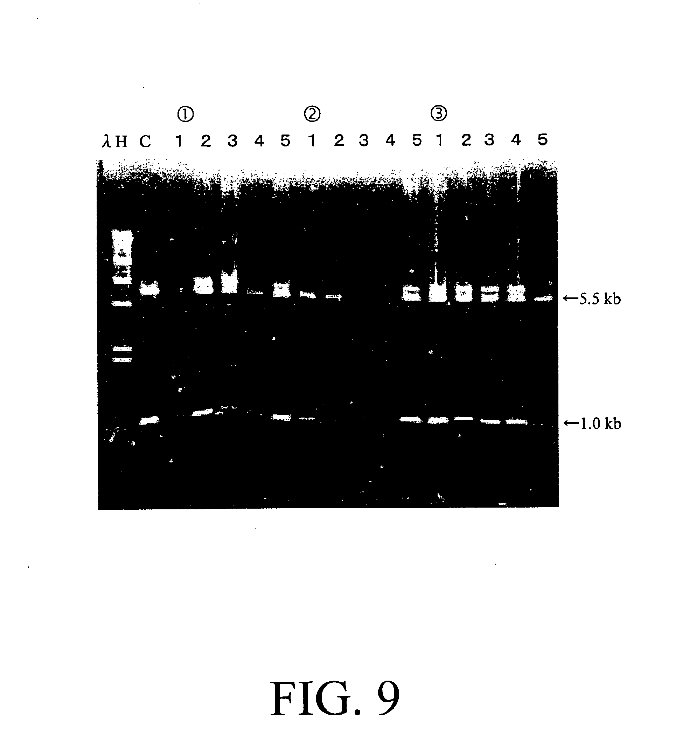

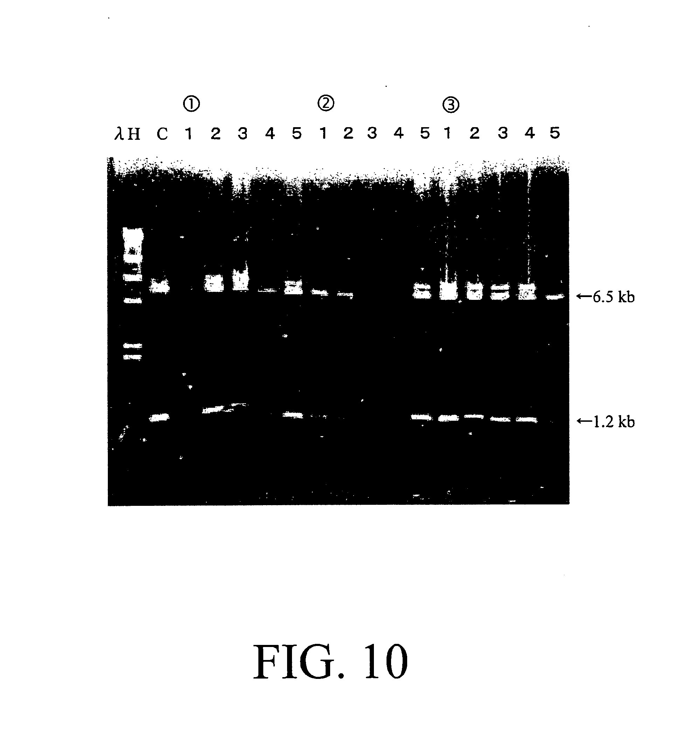

In order to confirm whether or not a plasmid was correctly incorporated into a transformant of an yeast used in Example A-1, mini-prep of the yeast was performed. For each plasmid, the mini-prep of the yeast was performed for three transformants. For each of the transformants obtained by transforming *Escherichia coli* with the resulting product, the mini-prep of *Escherichia coli* was performed in quintuple. The confirmation was made by cleavage with a restriction enzyme. In other words, 15 samples were evaluated for one plasmid. The transformant of the yeast was inoculated into 5 ml of an YPD liquid medium, and cultured overnight at 30°C, and the culture was centrifuged at 8000 rpm for 5 minutes. The precipitates were dissolved in 0.5 ml of 1 M sorbitol (pH 7.5), and the solution was transferred to an Eppendorf (registered trademark) tube. Thereto were added 3.6 µl of 2-mercaptoethanol and 20 µl of 2.5 mg/ml zymolyase 60,000, and the mixture was allowed to stand at 37°C for 60 minutes. After the mixture was centrifuged at 14,000 rpm for 20 seconds, the precipitates were dissolved in 0.5 ml of 50 mM Tris pH 7.4, 20 mM EDTA, and 50 µl of 10% SDS was added thereto while thoroughly mixing. The mixture was allowed to stand at 65°C for 30 minutes. Thereto was added 0.2 ml of 5M potassium acetate, and the mixture was allowed to stand on ice for 1 hour, and centrifuged at 14,000 rpm for 5 minutes. An upper layer was transferred to another Eppendorf (registered trademark) tube, and an equivolume of isopropanol was added thereto while mixing. The mixture was allowed to stand at room temperature for 5 minutes. After the mixture was centrifuged at 14,000 rpm for 10 seconds, the precipitates were washed with 70% ethanol, and the precipitates were dried, and dissolved in 300 µl of TE containing RNase. After the solution was allowed to stand at 37°C for 30 minutes, the solution was extracted twice with phenol, and extracted three times with ether, and the extract was subjected to ethanol precipitation. Thereafter, the precipitates were dissolved in 50 µl of TE. Thereafter, *Escherichia coli* was transformed with 5 µl of the prepared DNA, and plasmid DNA was prepared from the resulting transformant of *Escherichia coli* by mini-prep. After the plasmid DNA was cleaved with an appropriate restriction enzyme, the plasmid was confirmed by electrophoresis. The results for pESC-TRP are shown in Figure 9, and The results for pESC-TRP-tob are shown in Figure 10.

After the mini-prep of *Escherichia coli,* pESC-TRP was checked by cleavage with *Xba*I. As a result, there could be confirmed the presence of the bands at 5.5 kb and 1.0 kb in the same manner as the control except for the procedure. It could be confirmed from the results that the plasmid was almost perfectly transformed into the yeast for pESC-TRP.

After the mini-prep of *Escherichia coli,* pESC-TRP-*tob* was checked by cleavage with *Sal*I and *Bam*HI. Although some bands were weak and indistinguishable, the same bands at 6.5 kb and 1.2 kb as those of the control could be confirmed in almost all the cases. It could be confirmed from the results that the plasmid was almost perfectly transformed into the yeast also for pESC-TRP-*tob*.

### Example A-4 Expression 1 of hcaf1 Gene in Yeast S. cerevisiae

In this example, Caf protein was expressed in cells by introducing *hcaf1* gene into an yeast S. *cerevisiae* YPH500α strain, and the change in the growing state of the yeast caused thereby was observed. In other words, a plasmid for introducing *hcaf1* gene was constructed, an yeast was transformed with the constructed plasmid, and the growing state thereof was observed.

As a vector, pESC-URA was used for subcloning. In addition, as *hcaf1* gene, pcDNA3-*hcaf1* containing the gene (one in which *hcaf1-myc* gene was inserted into *Bam*HI*-Xho*I site of pcDNA3) was amplified, and the resulting gene was used.

### (1) Preparation of pESC-URA-hcaf1

*Escherichia coli* JM109 was transformed with pcDNA3*-hcaf1*, and plasmid DNA was prepared from the resulting transformant (medium-scale extraction). *Escherichia coli* was also transformed with pESC-URA, and plasmid DNA was prepared in the same manner (medium-scale extraction). The prepared plasmid pcDNA3*-hcaf1* was treated with *Bam*HI and *Xho*I, and DNA was collected by cut-out from a gel. Also, as to pESC-URA, the plasmid was treated with *Bam*HI and *Xho*I, and DNA was collected by cut-out from a gel. The ligation was carried out with *hcaf1* gene and a fragment of pESC-URA collected by these experimental procedures, and *Escherichia coli* was transformed with the resulting product. After the resulting transformant was confirmed by mini-prep, plasmid DNA was prepared (medium-scale extraction). A nucleotide sequence of the introduced gene was confirmed by sequencing from both sides of a gene-introduced portion.

### (2) Expression of Caf Protein in YPH500α Strain and Growth Experiment

An YPH500α strain was transformed with the prepared plasmid according to the lithium acetate method. The resulting transformant was subcultured on a different CSM-URA solid medium, and cultured at 30°C for 2 to 3 days, and thereby a transformant having no uracil-requiring property was screened. A colony was inoculated from the plate into 5 ml of a CSM-URA Glc liquid medium, and the colony was cultured at 30°C for 2 to 3 days while shaking. A 0.1 ml of culture medium each was subcultured on two 5 ml of CSM-URA Gal liquid media and one CSM-URA Glc liquid medium, and cultured at 30°C for 4 to 7 days while shaking. The KU values of the CSM-URA Gal liquid medium in which a Caf protein was expressed was determined every 2 hours during the culture, and the growing state of yeast was observed thereby. Separately, the protein was collected from another culture medium of CSM-URA Gal liquid medium, and expression of the Caf protein was confirmed by Western blotting. The yeast inoculated into the CSM-URA Glc liquid medium was cultured to an intermediate phase of a logarithmic growth phase, washed with 5 ml of the CSM-URA Gal liquid medium, and suspended. The yeast was cultured at 30°C for several hours, and thereafter protein was collected, and subjected to Western blotting.

### (3) Analysis of Growth Inhibition of YPH500α Strain by Expression of Caf Protein

A pESC vector, which is a protein expression vector for the yeast, has a GAL1 promoter, and expresses a desired gene only in the presence of galactose. In order to evaluate the change in the growing state of YPH500α strain by expression of *hcaf1* gene, the growth of the yeast was evaluated by first pre-culturing the yeast in a medium containing glucose, and thereafter subculturing the pre-culture in a medium containing galactose, thereby expressing *hcaf1* gene. The specific experimental method was carried out in the same manner as in Example A-1.

### Expression of hcaf1 Gene in YPH500α Strain

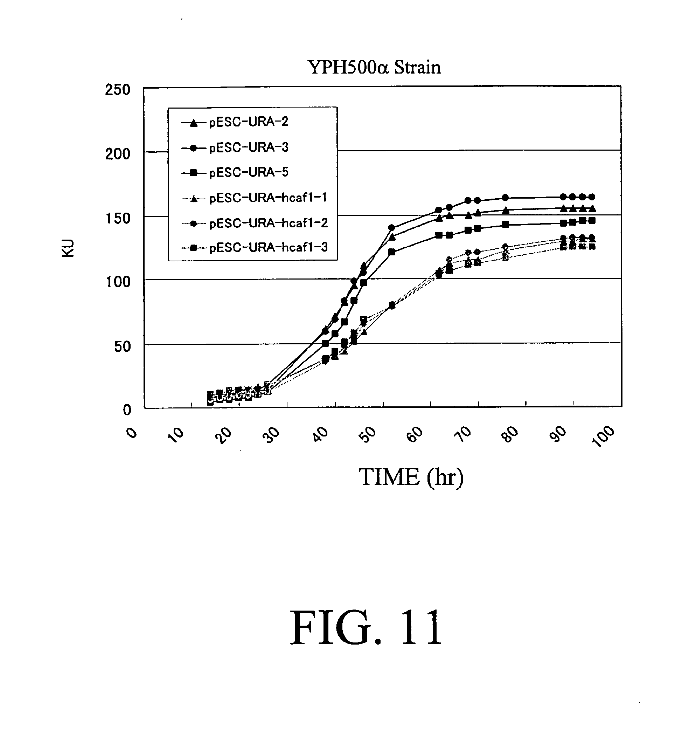

The results of observation of the growing state for the YPH500α strain (proliferation curve) are shown in Figure 11. Overall, although the difference in the growth between the control vector-introduced yeast and the *hcaf1*-introduced yeast was not as great, as compared to the case of tob gene, the growth inhibition was found in the transformant of an yeast into which *hcaf1* gene was introduced in the same manner.

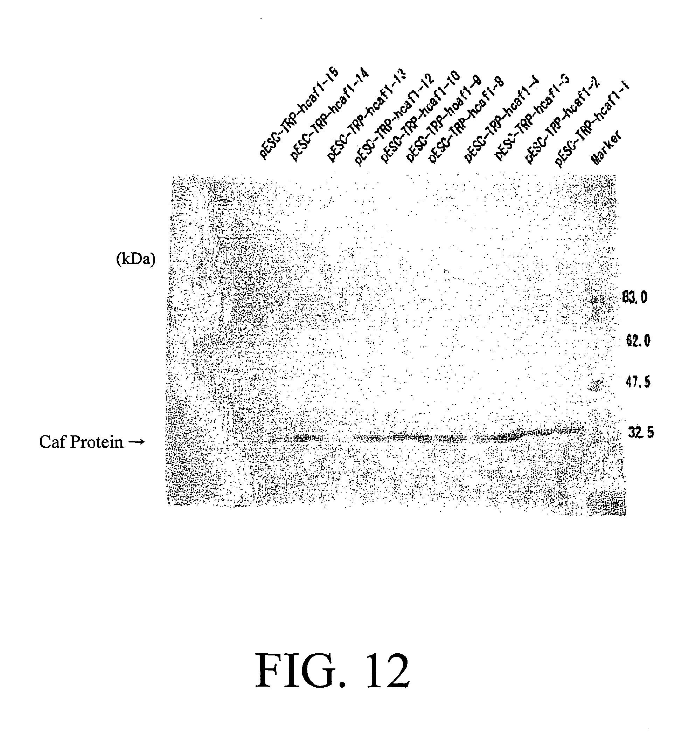

The culture was stopped for the CMS-URA Gal liquid medium previously cultured for Western blotting at a point where the yeast was cultured for 70 hours. The protein was collected according to the alkali-TCA method, and subjected to Western blotting. In addition, the CSM-URA Glc liquid medium previously cultured similarly for Western blotting was cultured to an intermediate phase of a logarithmic growth phase, and the culture medium was washed with and suspended in the CSM-URA Gal liquid medium. After the culture medium was cultured for 10 hours, the protein was collected, and subjected to Western blotting. The results are shown in Figure 12. The amount 50 µg of the protein was applied to a 7.5% acrylamide gel, and the protein was detected using a myc antibody as a primary antibody. It can be seen from the figure that the Caf protein was captured in almost all the samples.

During the Western blotting of Tob, the bands of Tob previously degraded by a protease in cells appeared other than the band for Tob. However, in this case, only the bands for Caf were obtained. It can be said from these results that Caf is a very stable protein. Similar results are shown also in an experiment performed with animal cells, and the results in the experiment for the yeast have the same tendencies as those for the animal cells.

### Example A-5 Expression 2 of hcaf1 Gene in Yeast S. cerevisiae

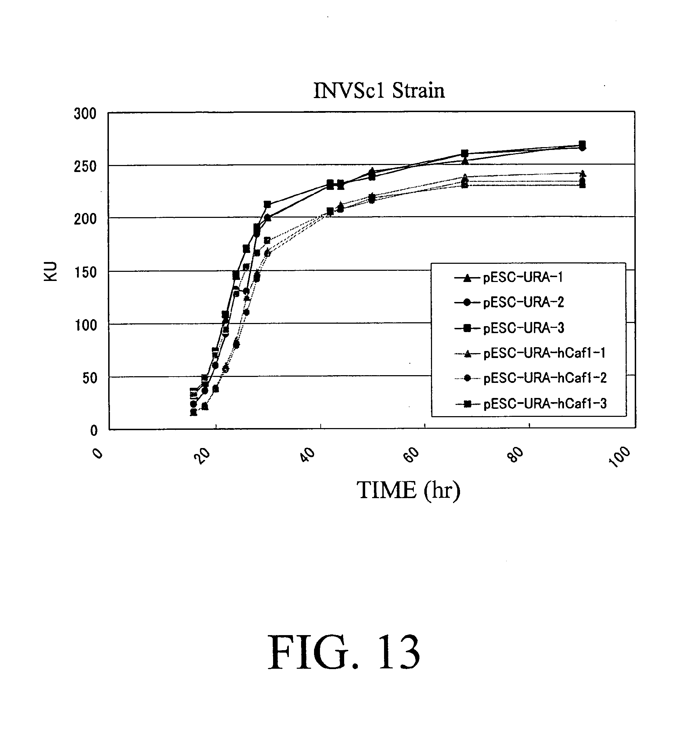

The growth was evaluated in the same manner as in Example A-4 by introducing *hcaf1* gene into an yeast *S*. *cerevisiae* INVSc1 strain. The results are shown in Figure 13. Although the degree of growth inhibition due to Caf is low as compared to that of the YPH500α strain, stable results were obtained with little scatter in growth.

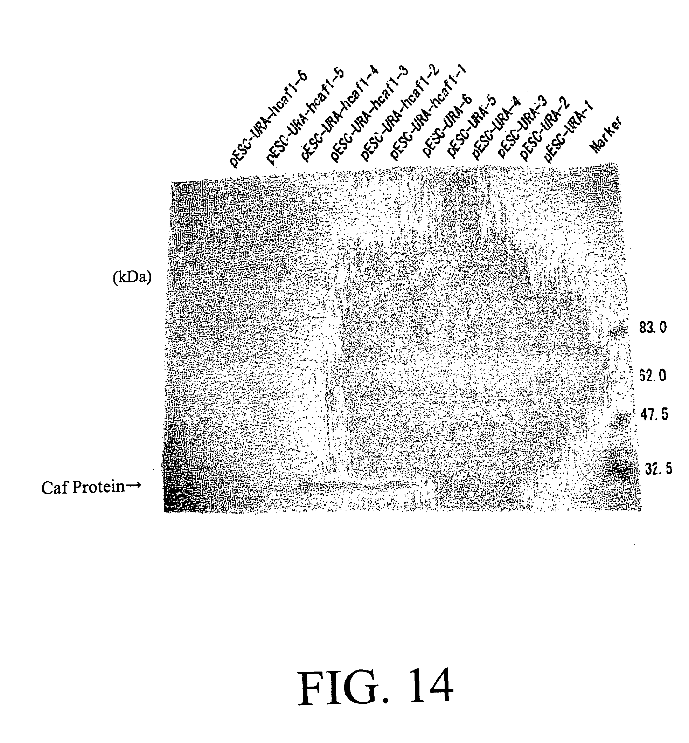

The culture was stopped for the CSM-URA Gal liquid medium previously cultured for Western blotting at a point where the yeast was cultured for 24 hours. The protein was collected according to the alkali-TCA method, and subjected to Western blotting. The results are shown in Figure 14. In the same manner as the previous case, 50 µg of the protein was applied to a 7.5% gel, and electrophoresed, and the protein was detected using the myc antibody as a primary antibody.

### Example A-6 Simultaneous Expression of tob Gene and hcaf1 Gene in Yeast S. cerevisiae

In this Example, the Tob protein and the Caf protein were coexpressed in cells by simultaneously introducing *tob* gene and *hcaf1* gene into an yeast *S*. *cerevisiae* INVSc1 strain, and the change in the growing state of the yeast caused thereby was observed. In other words, an yeast was transformed with the plasmid for introducing *tob* gene and the plasmid for introducing *hcaf1* gene previously constructed, and the growing state thereof was observed.

As a transformation vector, pESC-TRP-*tob* (one in which *tob* gene was inserted into *Bam*HI*-Sal*I site of pESC-TRP) and pESC-URA-*hcaf1* (one in which *hcaf1* gene was inserted into *Bam*HI-*Xho*I site of pESC-URA) were used.

### (1) Simultaneous Expression of Tob Protein and Caf Protein and Growth Experiment in INVSc1 Strain

An INVSc1 strain was transformed with the above-mentioned plasmid according to the lithium acetate method. The resulting transformant was subcultured on a different CSM-TRP-URA solid medium, and cultured at 30°C for 2 to 3 days, and thereby a transformant having both tryptophan-requiring and uracil-requiring properties was screened. A colony was inoculated from the plate into 5 ml of a CSM-TRP-URA Glc liquid medium, and the colony was cultured at 30°C for 2 to 3 days while shaking. Each 0.1 ml of the culture medium was subcultured on two 5 ml of CSM-TRP-URA Gal liquid media, and cultured at 30°C for 4 to 7 days while shaking. The KU values of the CSM-TRP-URA Gal liquid medium in which the Tob protein and the Caf protein were expressed were determined every several hours during the culture, and the growing state of the yeast was observed thereby. Separately, the protein was collected from another culture medium of CSM-TRP-URA Gal liquid medium, and expression of the Tob protein and the Caf protein was confirmed by Western blotting.

### (2) Analysis of Growth Inhibition of INVSc1 Strain by Simultaneous Expression of Tob Protein and Caf Protein

A pESC vector, which is a protein expression vector for the yeast, has a GAL1 promoter, and expresses a desired gene only in the presence of galactose. In order to evaluate the change in the growing state of INVSc1 strain by simultaneous expression of *tob* gene and *hcaf1* gene, the growth of the yeast was evaluated by first pre-culturing the yeast in a medium containing glucose, and thereafter subculturing in a medium containing galactose, thereby simultaneously expressing *tob* gene and *hcaf1* gene.

### Simultaneous Expression of tob Gene and hcaf1 Gene in INVSc1 Strain

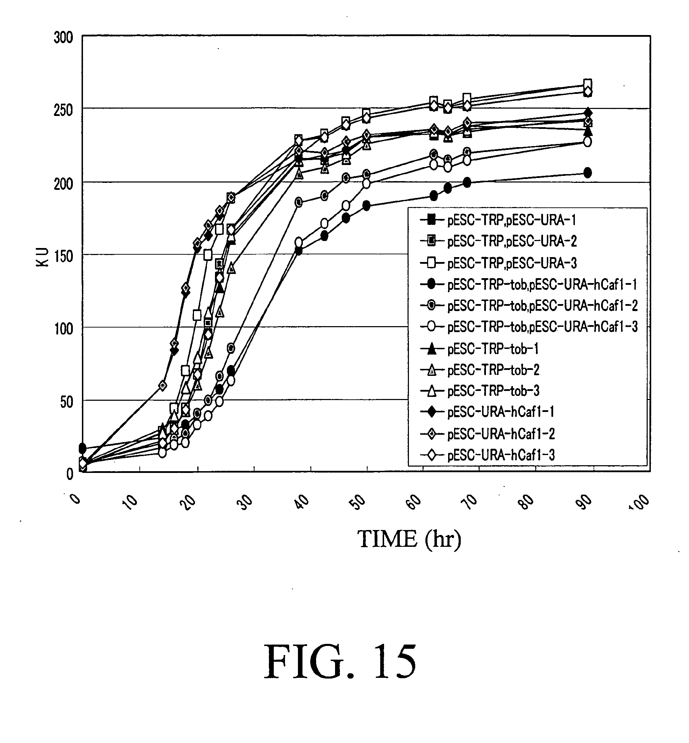

The KU values for the INVSc1 strain were plotted against the culture time, a proliferation curve was prepared. The results are shown in Figure 15.

Similar to the case where *tob* gene or *hcaf1* gene was alone introduced in the above-mentioned experiment of the INVSc1 strain, the growth inhibition was found as compared to that of the control, and its extent was larger than the case of the gene alone.

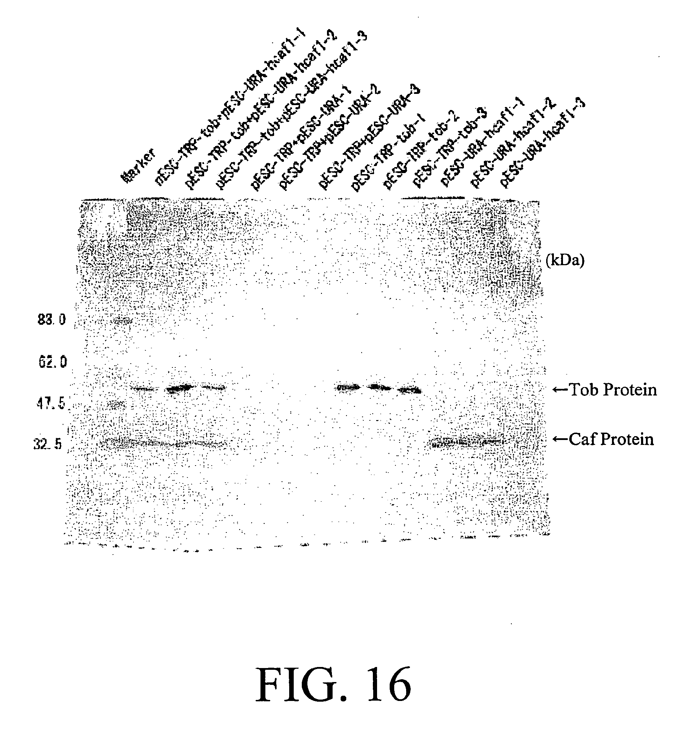

The results of Western blotting are shown in Figure 16. The amount 50 µg of the protein was applied to a 7.5% of acrylamide gel, and the protein was detected using a myc antibody as a primary antibody. It can be seen from the figure that the Tob protein and the Caf protein were clearly captured, respectively, and expression of each protein could be confirmed.

### (B) Examples in Yeast Having Aspiration Ability

The strains used in the following example are shown in Table 2.

**Table 2**

| Micro-organism | Strain | Genotypes |
|---|---|---|
| *E. coli* | JM109 | *hsd*R17(rK⁻, mK⁺), *rec*A1, *end*A1*, Δ(lac-pro*AB), *gyr*A96, *thi-*1, *rel*A1, *supE*44, F'[*tra*D36,*pro*AB, *lac*I^{q}, *lac*ZΔM15], e14^{*-*}(*McrA*^{*-*}) |
| *S*. *cerevisiae* | YPH500-12 | *MATα, ura3-52, lys2-801*^{*amber*}, *ade2-101*^{*ochre*}, *trp-Δ63, his3-1Δ200, leu2-Δ1, ρ*^{*0*} (or *ρ*^{*-*}) |

In addition, an aspiration ability-deficient strain (ρ⁻) of an yeast was acquired according to the following method.

### (1) Acquirement of Aspiration Ability-Deficient Strain (ρ⁻) of Yeast

An yeast parent strain (ρ⁺) was inoculated into 5 ml of an SD medium (0.67% Yeast Nitrogen Base w/o amino acid +2% glucose) with addition of 25 µg/ml ethidium bromide (EtBr), and the yeast was cultured until full growth at 30°C for 3 days. A 0.1 ml culture medium was taken from the culture, subcultured on a fresh SD medium added with EtBr, and cultured to full growth at 30°C for 3 days, and the culture diluted with sterile water. The dilution was plated on an YPD plate, and cultured at 30°C for 3 days. The resulting single colony was subcultured on an YPD plate and an YP glycerol plate, to confirm the acquirement of the aspiration ability-deficient strain (ρ-).

The media, the reagents and the general experimental methods used in the following example are the same as those described in the above-mentioned (A).

### Example B-1 Expression of tob (htob) Gene in Aspiration Ability-Deficient Strain of Yeast Saccharomyces cerevisiae

In this example, Tob protein was expressed in cells by introducing *tob* (*htob*) gene into an yeast *Saccharomyces cerevisiae* YPH500-12 strain (ρ⁻), and the change in the growing state of the yeast caused thereby was observed. In other words, a plasmid for introducing *tob* gene was constructed, the aspiration ability-deficient strain of the yeast obtained by the above-mentioned method was transformed with the constructed plasmid, and the growing state thereof was observed.

As a vector, pESC-TRP was used for subcloning. As *tob* gene, pME18S-*tob* containing the gene (one in which *tob* gene was inserted into EcoRI site of pME18S) was amplified, and the resulting gene was used.

### (1) Preparation of pESC-TRP-tob

*Escherichia coli* JM109 was transformed with pME18S-*tob*, and plasmid DNA was prepared (medium-scale extraction) from the resulting transformant. *Escherichia coli* was also transformed with pESC-TRP, and plasmid DNA was prepared in the same manner (medium-scale extraction). PCR was performed with the prepared plasmid pME18S-*tob* as a template using following primers
a forward primer:
   5'-cccggatcca tgcagcttga aatccaagta-3' (SEQ ID NO: 5), and
a reverse primer:
   5'-cccgtcgacg ttagccataa caggctggaa-3' (SEQ ID NO: 6)
to amplify *tob* gene. The resulting PCR product was treated with *Bam*HI and *Sal*I, and DNA was then collected by cut-out from the gel. As to pESC-TRP, after the treatment with *Bam*HI and *Sal*I, DNA was collected by cut-out from a gel. The ligation was carried out with *tob* gene and a fragment of pESC-TRP collected by these experimental procedures, and *Escherichia coli* was transformed with the resulting product. After the resulting transformant was confirmed by a mini-prep, plasmid DNA was prepared (medium-scale extraction).

### (2) Expression of Tob Protein and Growth Experiment in YPH500-12 Strain (ρ⁻)

An YPH500-12 strain (ρ⁻) was transformed with the prepared plasmid according to the lithium acetate method. The resulting transformant was subcultured on a different CSM-TRP solid medium, and cultured at 30°C for 2 to 3 days, and thereby a tryptophan-requiring transformant was screened. A colony was inoculated from the plate into 5 ml of a CSM-TRP Glc(2%) medium, and the colony was pre-pre-cultured. At a point where the colony was pre-pre-cultured for 162 hours at 30°C, a 0.1 ml culture medium each was taken from the culture medium, and subcultured on 5 ml of a CSM-TRP Raf (2%) medium. After the pre-culture was further carried out for 122 hours, a 0.1 ml culture medium each was taken from the culture, and subcultured on two 5 ml of CSM-TRP Raf(2%) media and CSM-TRP Raf (2%) + Gal (2%) media, and the growth was observed. The protein was collected for Western blotting at a point where the yeast was cultured for 69 hours. The amount 50 µg of the protein was electrophoresed using a 10% acrylamide gel, and then transferred onto a nitrocellulose membrane. The myc antibody was used for detecting the Tob protein. The myc antibody is an antibody specific for myc tag added to a C-terminal of the Tob protein.

### (3) Analysis of Lowering in Growth of YPH500-12 strain (ρ⁻) by Expression of Tob Protein

A pESC vector, which is a protein expression vector for an yeast, has a GAL1 promoter, and expresses a desired gene only in the presence of galactose. Therefore, a transformed yeast was cultured in the presence of galactose to induce expression of the Tob protein.

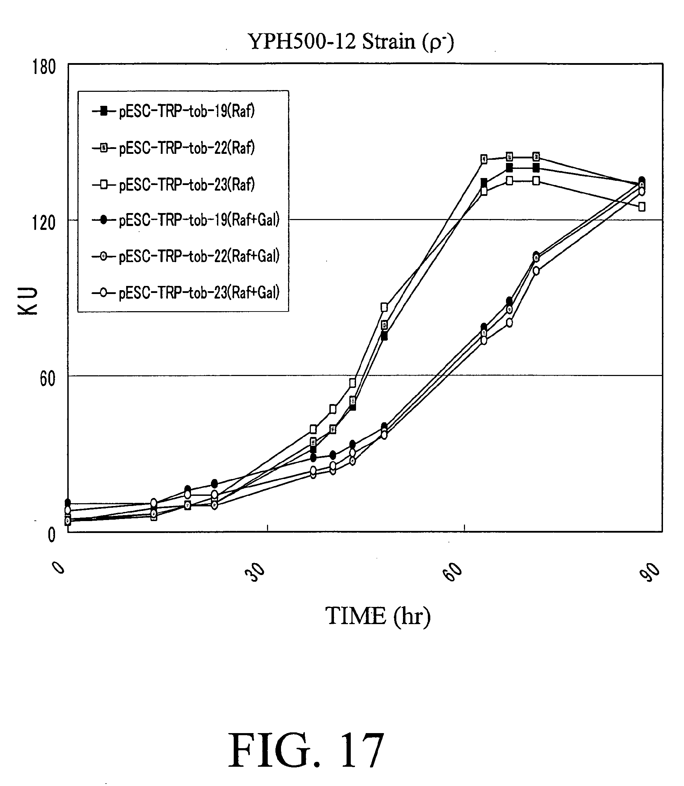

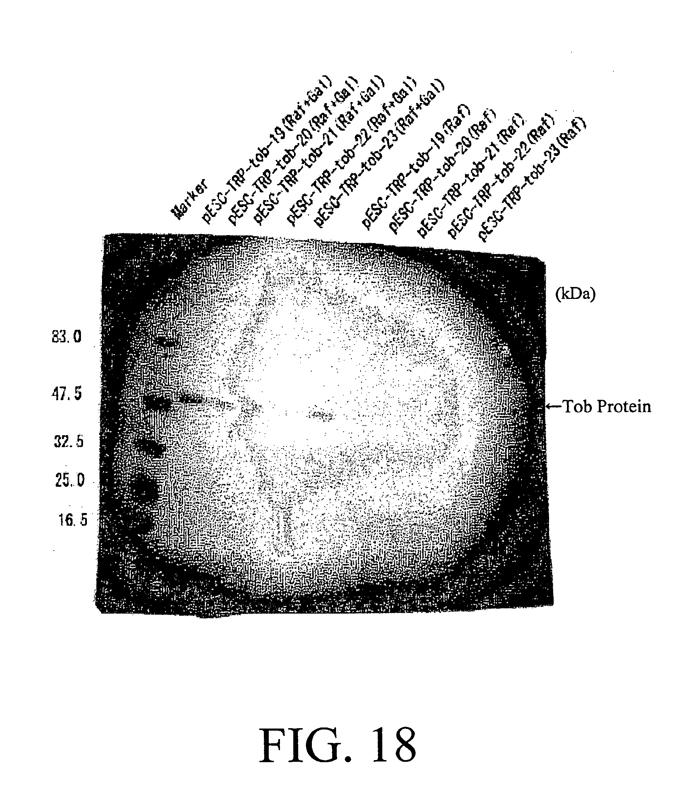

The KU values for the YPH500-12 strain (ρ⁻) were plotted against the culture time, and a proliferation curve was prepared. The proliferation curve is shown in Figure 17. The results of Western blotting are shown in Figure 18.

It can be seen from Figure 17 that each of the transformed yeasts generally shows uniform growth without variance in a non-expression state (Raf) and in an expression state (Raf + Gal) of the Tob protein. In addition, it can be seen that the growth is clearly lowered in the expression state of the Tob protein as compared to that of the non-expression state of the protein. In other words, when a generation time and a proliferation rate are obtained, it can be determined that the growth is lowered in pESC-TRP-tob-19,22 or 23 (Raf + Gal), because the generation time is longer, and the proliferation rate is lower in pESC-TRP-*tob-*19, 22 or 23 (Raf + Gal), as compared to pESC-TRP-*tob*-19, 22 or 23 (Raf). In addition, as shown in Figure 18, the expressed Tob protein was captured in the yeast in the expression state of the Tob protein.

### Example B-2 Expression of caf (hcaf1) Gene in Aspiration Ability-Deficient Strain of Yeast S. cerevisiae

In this example, Caf protein was expressed in cells by introducing *caf* (*hcaf1*) gene into an yeast *S. cerevisiae* YPH500-12 strain (ρ⁻), and the change in the growing state of yeast caused thereby was observed. In other words, a plasmid for introducing *hcaf1* gene was constructed, the aspiration ability-deficient strain of the yeast obtained by the above-mentioned method was transformed with the constructed plasmid, and the growing state thereof was observed.

As a vector, pESC-URA was used for subcloning. As *hcaf1* gene, pcDNA3-*hcaf1* containing the gene (one in which *hcaf1-myc* gene was inserted into *Bam*HI*-Xho*I site of pcDNA3) was amplified, and the resulting gene was used.

### (1) Preparation of pESC-URA-hcaf1

*Escherichia coli* JM109 was transformed with pcDNA3-*hcaf1*, and plasmid DNA was prepared (medium-scale extraction) from the resulting transformant. *Escherichia coli* was also transformed with pESC-URA, and plasmid DNA was prepared in the same manner (medium-scale extraction). The prepared plasmid pcDNA3*-hcaf1* was treated with *Bam*HI and *Xho*I*,* and DNA was collected by cut-out from the gel. As to pESC-URA, after the treatment with *Bam*HI and *XhoI,* DNA was collected by cut-out from the gel. The ligation was carried out with *hcaf1* gene and a fragment of pESC-URA collected by these experimental procedures, and *Escherichia coli* was transformed with the resulting product. After the resulting transformant was confirmed by a mini-prep, plasmid DNA was prepared (medium-scale extraction). A nucleotide sequence of the introduced gene was confirmed by sequencing from both sides of a gene-introduced portion.

### (2) Expression of Caf Protein and Growth Experiment in YPH500-12 Strain (ρ⁻)

Expression of the Caf protein and the growth experiment were carried out in the same manner as in Example B-1. The CSM-URA medium was used as a medium. The protein for Western blotting was collected at a point where the yeast was cultured for 45 hours.

### (3) Analysis of Lowering in Growth of YPH500-12 Strain (ρ⁻) by Expression of Caf Protein

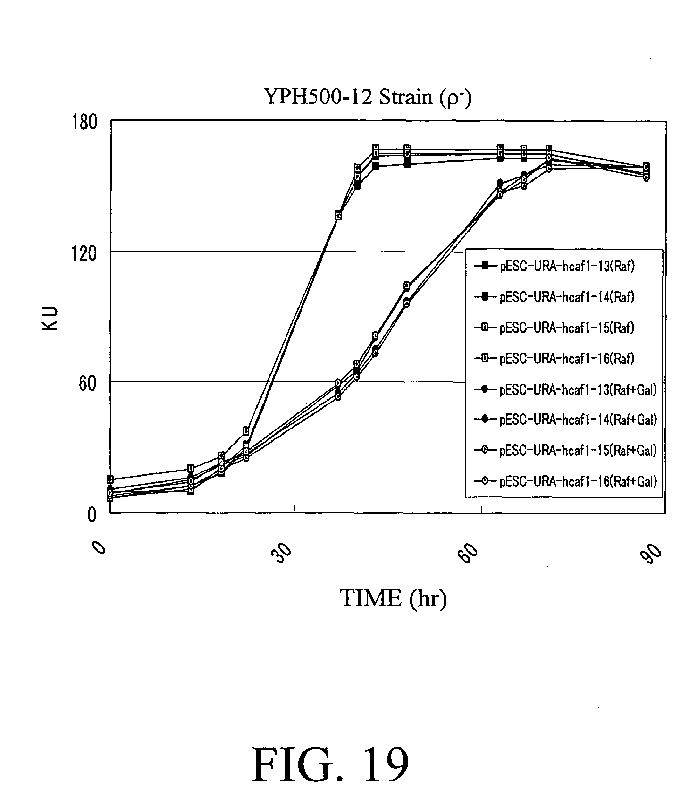

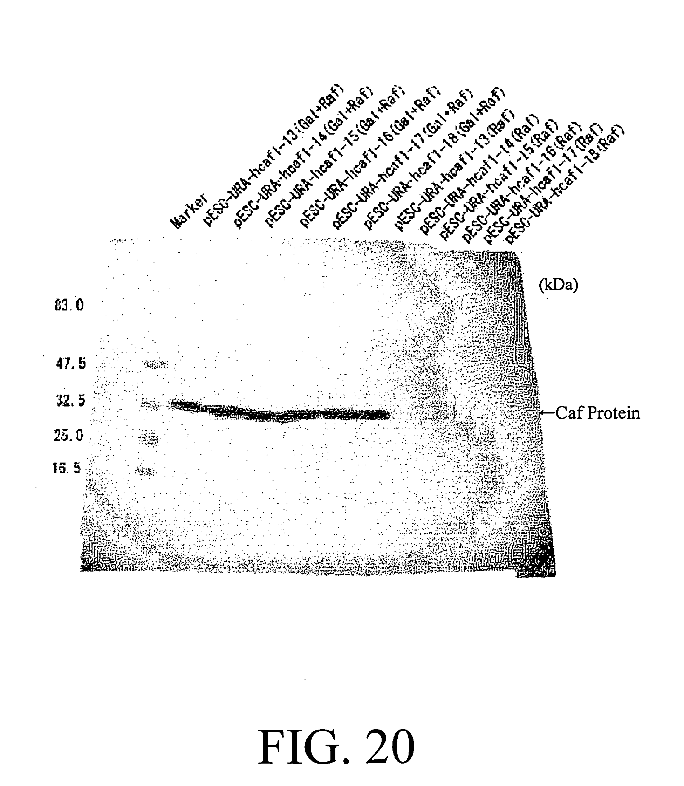

A proliferation curve for the YPH500-12 strain (ρ-) was prepared in the same manner as in Example B-1. The proliferation curve is shown in Figure 19. The results of Western blotting are shown in Figure 20.

It can be seen from Figure 19 that each of the transformed yeasts generally shows uniform growth without variance in non-expression state and in expression state of the Caf protein. In addition, it can be seen that the growth is clearly lowered in the expression state of the Caf protein as compared to that of the non-expression state of the protein. In other words, when a generation time and a proliferation rate are obtained, it can be determined that the growth is lowered in pESC-URA-*hcaf1* -13, 14, 15, or 16 (Raf + Gal), because the generation time is longer, and the proliferation rate is lower in pESC-URA-*hcaf1-*13,14,15 or 16 (Raf + Gal), as compared to pESC-URA-*hcaf1*-13,14, 15 or 16 (Raf). In addition, as shown in Figure 20, the expressed Caf protein was captured in the yeast in the expression state of the Caf protein.

### Example B-3 Coexpression of tob Gene and hcaf1 Gene in Aspiration Ability-Deficient Strain of Yeast S. cerevisiae

In this example, the Tob protein and the Caf protein were coexpressed in cells by simultaneously introducing *tob (htob)* gene and *caf* (*hcaf1*) gene into an yeast *S. cerevisiae* YPH500-12 strain (ρ⁻), and the change in the growing state of yeast caused thereby was observed. In other words, the aspiration ability-deficient strain of the yeast obtained by the above-mentioned method was transformed with the plasmid for introducing *tob* gene and the plasmid for introducing *hcaf1* gene constructed previously, and the growing state thereof was observed.

### (1) Expression of Tob protein and Caf protein and Growth Experiment in YPH500-12 Strain (ρ⁻)

Coexpression of the Tob protein and the Caf protein and the growth experiment were carried out in the same manner as in Example B-1 except that the YPH500-12 strain (ρ') was transformed simultaneously with pESC-TRP-*tob* and pESC-URA-*hcaf1*. The protein for Western blotting was collected at a point where the yeast was cultured for 144 hours.

### (2) Analysis of Lowering in Growth of YPH500-12 Strain (ρ⁻) by Coexpression of Tob Protein and Caf Protein

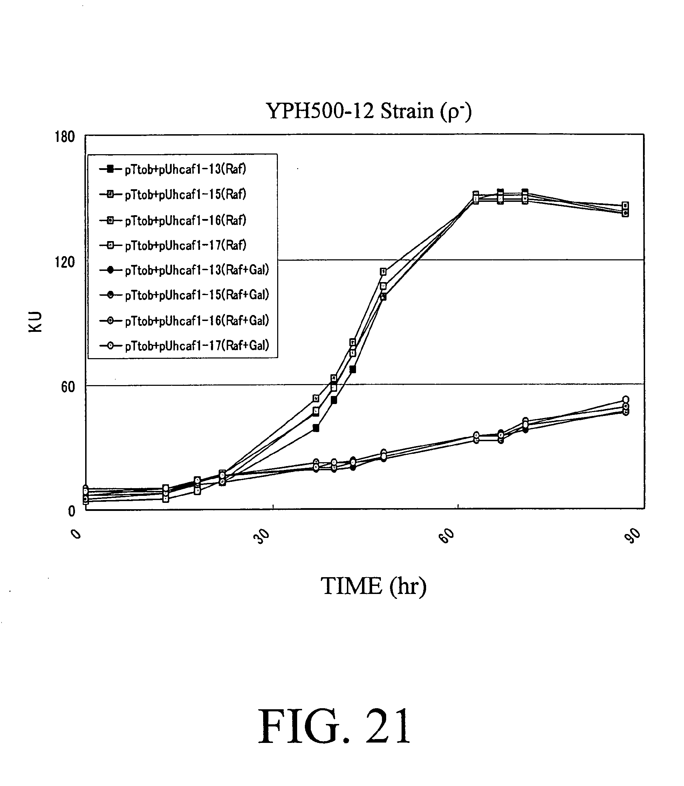

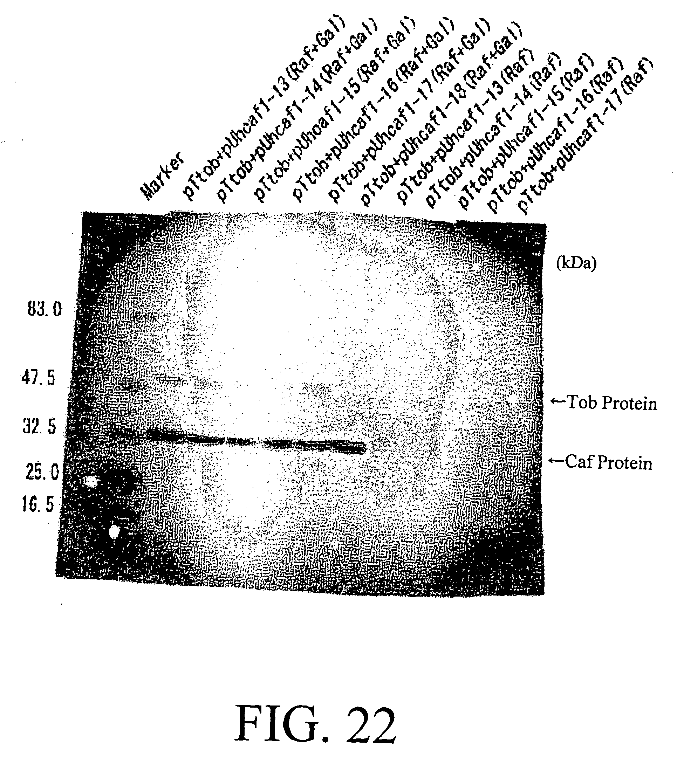

A proliferation curve of an YPH500-12 strain (ρ⁻) was prepared in the same manner as in Example B-1. The proliferation curve is shown in Figure 21. The results of Western blotting are shown in Figure 22. In the figure, pESC-TRP-*tob* is abbreviated as pTtob, and pESC-URA-*hcaf1* is abbreviated as pUhcaf1.

It can be seen from Figure 21 that each of the transformed yeasts generally shows uniform growth without variance in non-expression state and in expression state of the above-mentioned both the proteins. In addition, it can be seen that the growth is clearly lowered in the expression state of both the proteins as compared to those of the non-expression state of these proteins. In other words, when a generation time and a proliferation rate are obtained, it can be determined that the growth is lowered in pTtob + pUhcaf1-13, 15, 16 or 17 (Raf + Gal), because the generation time is longer, and the proliferation rate is lower in pTtob+pUhcaf1-13, 15, 16 or 17 (Raf + Gal), as compared to pTtob+pUhcaf1-13, 15, 16 or 17 (Raf). In addition, when the Tob protein and the Caf protein are coexpressed in the yeast, it can be seen that the lowering in growth of the yeast is enhanced as compared to the case where the proteins are expressed alone. As shown in Figure 22, the expressed Tob protein and Caf protein were captured in the yeast in the expression state of both the proteins.

### Example B-4 Expression of lck Gene in Aspiration Ability-Deficient Strain of Yeast S. cerevisiae

In this example, Lck protein (tyrosine kinase) was expressed in cells by introducing *lck* gene into an yeast *S*. *cerevisiae* YPH500-12 strain (ρ-), and the change in the growing state of yeast caused thereby was observed. In other words, a plasmid for introducing *lck* gene was constructed, the aspiration ability-deficient strain of the yeast obtained by the above-mentioned method was transformed with the constructed plasmid, and the growing state thereof was observed.

As a vector, pESC-URA was used for subcloning. As *lck* gene, pME18SH-*lck* containing the gene (one in which *lck* gene was inserted into HindIII site of pME18SH [a vector obtained by disrupting the original HindIII site of pME18S, and thereafter altering EcoRI site to HindIII site]) was amplified, and the resulting gene was used.

### (1) Preparation of pUlck

*Escherichia coli* JM109 was transformed with pME18SH-lck, and plasmid DNA was prepared (medium-scale extraction) from the resulting transformant. *Escherichia coli* was also transformed with pESC-URA, and plasmid DNA was prepared in the same manner (medium-scale extraction). PCR was performed with the prepared pME18SH-*lck* as a template using a forward primer:
5'- atgggctgtg gctgcagctc a-3' (SEQ ID NO: 7), and
a reverse primer:
5'- cccgtcgaca ggctgaggct ggtactggcc-3' (SEQ ID NO: 8) to amplify *lck* gene. The resulting PCR product was treated with *Sal*I, a terminal was then phosphorylated with kinase, and DNA was collected by cut-out from the gel. After pESC-URA was treated with *Bam*HI, a terminal was blunt-ended with a Klenow enzyme. This was treated with *Sal*I, and DNA was collected by cut-out form a gel. The ligation was carried out with *lck* gene and a fragment of pESC-URA collected by these experimental procedures, and *Escherichia coli* was transformed with the resulting product. After the resulting transformant was confirmed by a mini-prep, plasmid DNA was prepared (medium-scale extraction). By sequencing both ends of a gene-introducing portion, a nucleotide sequence of an introduced gene was confirmed.

### (2) Expression of Lck protein and Growth Experiment in YPH500-12 Strain (ρ⁻)

An YPH500-12 strain (ρ⁻) was transformed with the prepared plasmid according to the lithium acetate method. The resulting transformant was subcultured on a different CSM-URA solid medium, and cultured at 30°C for 2 to 3 days, and thereby a transformant having no uracil-requiring property was screened. A colony was inoculated from the plate into 5 ml of a CSM-URA Raf (2%) medium, and pre-cultured at 30°C for 96 hours. A 0.1 ml culture medium was taken from the culture, and subcultured on two 5 ml of CSM-URA Raf (2%) media and CSM-URA Raf (2%) + Gal (2%) media, respectively, and the growth was observed. The protein for Western blotting was collected at a point where the colony was cultured for 47 hours. The amount 50 µg of the protein was electrophoresed using a 7.5% acrylamide gel, and thereafter transferred onto a nitrocellulose membrane. The myc antibody was used for detecting the Lck protein.

### (3) Analysis of Lowering in Growth of YPH500-12 strain (ρ⁻) by Expression of Lck Protein

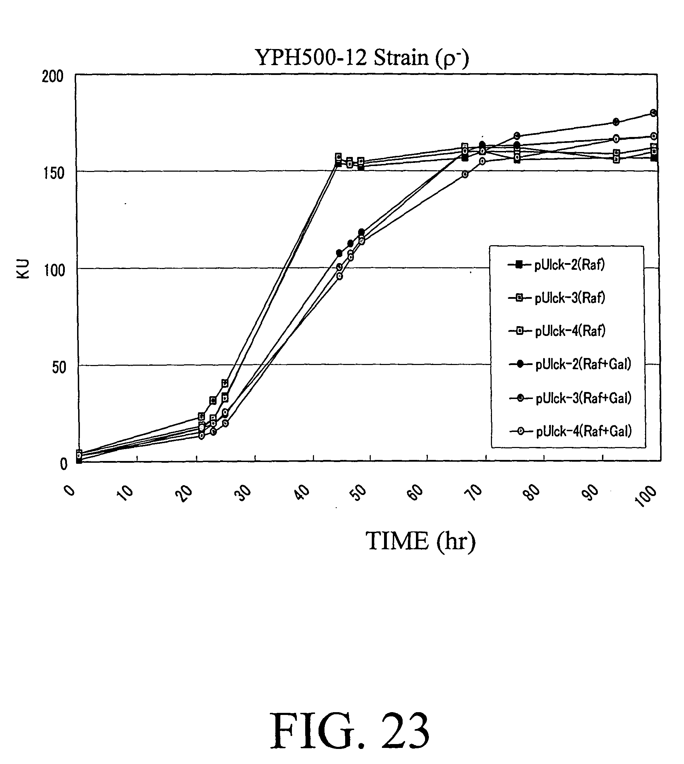

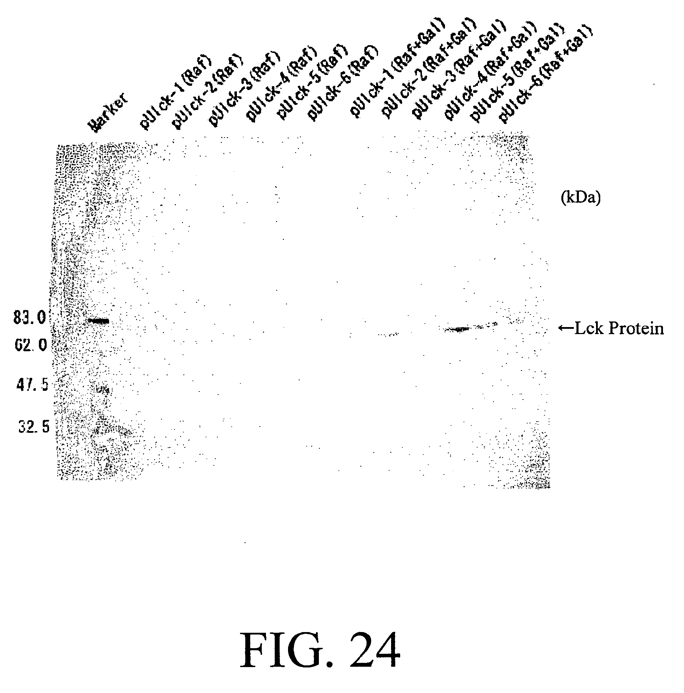

A proliferation curve of the YPH500-12 strain (ρ⁻) was prepared in the same manner as in Example B-1. The proliferation curve is shown in Figure 23. The results of Western blotting are shown in Figure 24.

It can be seen from Figure 23 that each of the transformed yeasts generally shows uniform growth without variance in a non-expression state and in an expression state of the Lck protein. In addition, it can be seen that the growth is clearly lowered in the expression state of Lck protein as compared to that of the non-expression state of the protein. In other words, when a generation time and a proliferation rate are obtained, it can be determined that the growth was lowered with pUlck-2, 3 or 4 (Raf + Gal), because the generation time is longer, and the proliferation rate is lower with pUlck-2, 3 or 4 (Raf + Gal), as compared to pUlck-2, 3 or 4 (Raf). In addition, as shown in Figure 24, the expressed Lck protein was captured in the yeast in the expression state of the Lck protein.

### Example B-5 Expression of lckYF Gene in Aspiration Ability-Deficient Strain of Yeast S. cerevisiae, and Reversal of Growth Inhibition by Addition of PP2

In this example, LckYF protein (Src-type tyrosine kinase) was expressed in cells by introducing *lckYF* gene into an yeast *S*. *cerevisiae* YPH500-12 strain (ρ⁻), and the change in the growing state of the yeast caused thereby was observed. At the same time, the change in the growing state of an yeast to which PP2 (4-amino-5-(4-chlorophenyl)-7-(*t*-butyl)pyrazolo[3,4-d]pyrimidine) (manufactured by CALBIOCHEM), which is a representative Src-type tyrosine kinase selective inhibitor, was previously added during the growth of the yeast expressing the LckYF protein, was observed. In other words, a plasmid for introducing *lckYF* gene was constructed, the aspiration ability-deficient strain of the yeast obtained by the above-mentioned method was transformed with the constructed plasmid, and the growing state thereof was observed. At the same time, the growing state of the transformed yeast to which the inhibitor PP2 of the LckYF protein, an expression product of *lckYF* gene, was previously added during the culture was observed.

The LckYF protein is an activated-type protein prepared by substituting 505th tyrosine (Tyr505) of the Lck protein with phenylalanine. It has been known that the activity of the Lck protein is lowered by phosphorylation of Tyr505. Therefore, an activated protein of which phosphorylation is inhibited by substituting Tyr505 with phenylalanine is the LckYF protein.

As a vector, pESC-URA was used for subcloning. Also, as *lckYf* gene, pME-LckYF containing the gene (one in which *lckYF* gene was inserted into a HindIII site of pME18SH [a vector obtained by disrupting the original HindIII site of pME18S, and altering EcoRI site thereof to HindIII site]) was amplified, and the resulting gene was used. In addition, as *lck* gene, pME-Lck containing the gene (one in which *lck* gene was inserted into HindIII site of pME18SH) was amplified, and the resulting gene was used.

### (1) Preparation of pESC-URA-lckYF

PCR was performed with pME-Lck as a template using
a forward primer:
   5'-atgggctgtg gctgcagctc a-3' (SEQ ID NO: 7), and
a reverse primer:
   5'-cccgtcgaca ggctgaggct ggtactggcc-3' (SEQ ID NO: 8)
to amplify *lck* gene. The resulting PCR product was treated with *Sal*I, a terminal was phosphorylated with kinase, and thereafter DNA was collected by cut-out from the gel. After pESC-URA was treated with *Bam*HI, terminal DNA was blunt-ended with a Klenow enzyme. Thereafter, after the treatment with *Sal*I, DNA was collected by cut-out from the gel. The ligation was carried out with a fragment of *lck* gene and a fragment of pESC-URA, and *Escherichia coli* was transformed with the resulting product. After the resulting transformant was confirmed by a mini-prep, plasmid DNA was prepared (medium-scale extraction). A nucleotide sequence of the introduced gene was confirmed by sequencing from both sides of a gene-introduced portion. The constructed plasmid was named as pESC-URA-*lck*.

Thereafter, PCR was performed with pME-LckYF as a template using
a forward primer:
   5'-atgggctgtg gctgcagctc a-3' (SEQ ID NO: 7), and
a reverse primer:
   5'-cccgtcgaca ggctgaggct gaaactggcc-3' (SEQ ID NO: 9)
to amplify *lckYF* gene. The resulting PCR product was treated with *Sma*I and *Sal*I, and DNA was collected by cut-out from the gel. After pESC-URA-*lck* was treated with *Sma*I and *Sal*I, DNA was collected by cut-out from the gel. The ligation was carried out with a fragment of *lckYF* gene and a fragment of pESC-URA-lck, and *Escherichia coli* was transformed with the resulting product. After the resulting transformant was confirmed by a mini-prep, plasmid DNA was prepared (medium-scale extraction). A nucleotide sequence of the introduced gene was confirmed by sequencing from both sides of a gene-introduced portion. The constructed plasmid was named as pESC-URA-*lckYF*.

### (2) Expression of LckYF Protein and Growth Experiment in YPH500-12 Strain (ρ⁻)

An YPH500-12 strain (ρ⁻) was transformed with the prepared plasmid according to the lithium acetate method. The resulting transformant was subcultured on a different CSM-URA solid medium, and cultured at 30°C for 2 to 3 days, and thereby a transformant having no uracil-requiring property was screened. A colony was inoculated from the plate into 5 ml of a CSM-URA Raf (2%) medium, and pre-cultured at 30°C for 3 days. A 0.1 ml culture medium was taken from the culture, and subcultured on 5 ml of a CSM-URA Raf (2%) + Gal (2%) medium to which PP2 was previously added so as to give a final concentration of 0, 1 or 5 µM, and the growth was observed. The protein for Western blotting was collected at a point where the colony was cultured for 95 hours. The amount 50 µg of the protein was electrophoresed using a 7.5% acrylamide gel, and then transferred onto a nitrocellulose membrane. In the detection of the LckYF protein, a 4G10 antibody, which is an anti-phosphorylated tyrosine antibody, was used.

### (3) Analysis of Lowering in Growth of YPH500-12 Strain (ρ⁻) by Expression of Lck Protein and Suppression of Lowering in Growth by Addition of PP2

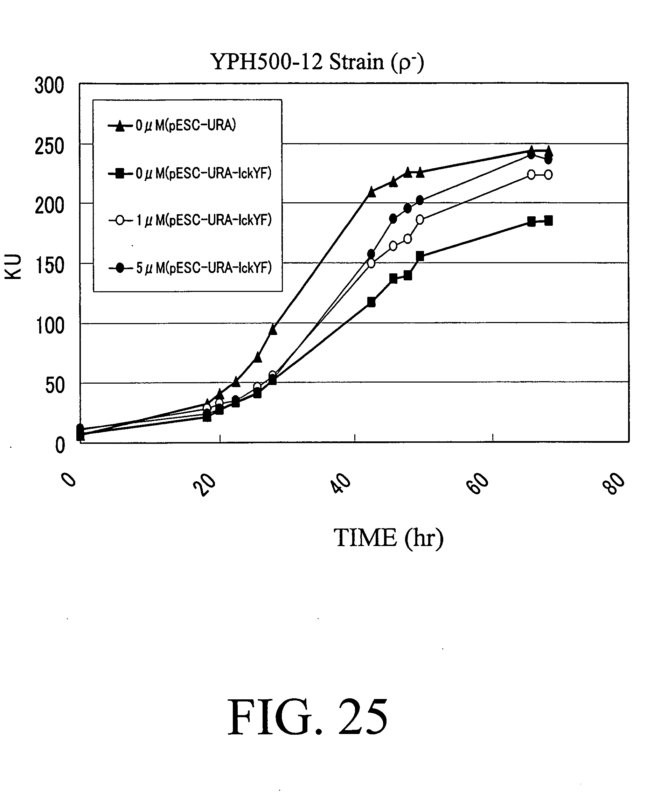

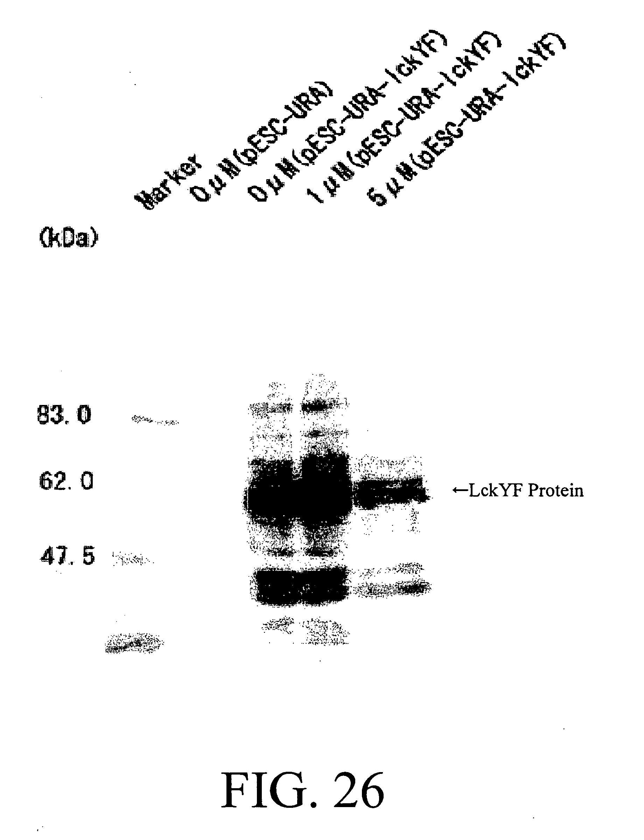

A proliferation curve of an YPH500-12 strain (ρ⁻) was prepared in the same manner as in Example B-1. The proliferation curve is shown in Figure 25. The results of Western blotting are shown in Figure 26.

It can be seen from Figure 25 that each of the transformed yeasts generally shows uniform growth without variance in a non-expression state and in an expression state of the LckYF protein. In addition, it can be seen that growth is clearly lowered in the expression state of the LckYF protein, as compared to that of the non-expression state of the protein. Further, it can be seen that the lowering in growth found in the expression state of LckYF protein is suppressed in a dose-dependent manner by the addition of PP2. In other words, when a generation time and a proliferation rate are obtained, it can be determined that the growth is lowered in culture at 0 µM (pESC-URA-lckYF), since a generation time is longer, and a proliferation rate is lower in 0 µM (pESC-URA-lckYF), as compared to culture at 0 µM (pESC-URA). In addition, in culture at 1 or 5 µM (pESC-URA-*lckYF*) with addition of PP2, the generation time and the proliferation rate fall between those for culture at 0 µM (pESC-URA) and culture at 0 µM (pESC-URA-*lckYF*), so that with the increase in the addition concentration, the generation time becomes shorter, and the proliferation rate is higher. Therefore, it can be determined that the lowering in growth accompanied with expression of the LckYF protein is suppressed in a dose-dependent manner, in other words, the growth is improved. In addition, as shown in Figure 26, the protein of an yeast which was previously phosphorylated by the expressed LckYF protein and the LckYF protein themselves were captured in the yeast in the expression state of the LckYF protein. In addition, as to culture at 5 µM (pESC-URA-*lckYF*) in which remarkable recovery of the growth was found by the addition of PP2, the lowering in the expression level was found, as compared to the case without addition of PP2, or culture at 1 µM (pESC-URA-*lckYF*). The results show that the activity of the LckYF protein was suppressed by the addition of PP2.

### Example B-6 Expression of tob (htob) Gene Simultaneously Using Four Kinds of Expression Vectors in Aspiration Ability-Deficient Strain of Yeast Saccharomyces cerevisiae

In this example, Tob protein was expressed in cells by introducing a *tob* (*htob*) gene into an yeast *Saccharomyces cerevisiae* YPH500-12 strain (ρ⁻) simultaneously using four kinds of expression vectors, and the change in the growing state of the yeast caused thereby was observed. In other words, four kinds of plasmids for introducing *tob* gene were constructed, the aspiration ability-deficient strain of yeast obtained by the above-mentioned method was transformed simultaneously with the constructed four kinds of plasmids, and the growing state thereof was observed.

As a vector, pESC-TRP, pESC-URA, pESC-LUE, and pESC-HIS were used for subcloning. In addition, as *tob* gene, pME18S-Tob containing the gene (one in which *tob* gene was inserted into EcoRI site of pME18S) was amplified, and the resulting gene was used.

### (1) Preparation of pESC-TRP-tob

pESC-*TRP-tob* was prepared in same manner as in Example B-1.

### (2) Preparation of pESC-TRP-tob

*Escherichia coli* JM109 was transformed with pME18S-Tob, and plasmid DNA was prepared (medium-scale extraction) from the resulting transformant. *Escherichia coli* was also transformed with pESC-TRP, and plasmid DNA was prepared in the same manner (medium-scale extraction). PCR was performed with the prepared plasmid pME18S-Tob as a template using
a forward primer:
   5'-cccggatcca tgcagcttga aatccaagta-3' (SEQ ID NO: 5), and
a reverse primer:
   5'-cccgtcgacg ttagccataa caggctggaa-3' (SEQ ID NO: 6)
to amplify *tob* gene. The resulting PCR product was treated with *Bam*HI and *Sal*I, and DNA was then collected by cut-out from the gel. As to pESC-TRP, after the treatment with *Bam*HI and *Sal*I, DNA was collected by cut-out from the gel. The ligation was carried out with *tob* gene and a fragment of pESC-TRP collected by these experimental procedures, and *Escherichia coli* was transformed with the resulting product. After the resulting transformant was confirmed by a mini-prep, plasmid DNA was prepared (medium-scale extraction).

### (3) Preparation of pESC-URA-tob

pESC-TRP-*tob* was treated with *Bam*HI and *Sal*I, and DNA was then collected by cut-out from the gel. As to pESC-URA, after the treatment with *Bam*HI and *Sal*I, DNA was collected by cut-out from the gel. The ligation was carried out with *tob* gene and a fragment of pESC-URA collected by these experimental procedures, and *Escherichia coli* was transformed with the resulting product. After the resulting transformant was confirmed by a mini-prep, plasmid DNA was prepared (medium-scale extraction).

### (4) Preparation of pESC-LUE-tob

pESC-TRP-*tob* was treated with *Bam*HI and *Sal*I, and DNA was then collected by cut-out from the gel. As for pESC-LUE, after the treatment with *Bam*HI and *Sal*I, DNA was collected by cut-out from the gel. The ligation was carried out with *tob* gene and a fragment of pESC-LUE collected by these experimental procedures, and *Escherichia coli* was transformed with the resulting product. After the resulting transformant was confirmed by a mini-prep, plasmid DNA was prepared (medium-scale extraction).

### (5) Preparation of pESC-HIS-tob

pESC-TRP-*tob* was treated with *Bam*HI and *Sal*I, and DNA was then collected by cut-out from the gel. As for pESC-HIS, after the treatment with *Bam*HI and *Sal*I, DNA was collected by cut-out from the gel. The ligation was carried out with *tob* gene and a fragment of pESC-HIS collected by these experimental procedures, and *Escherichia coli* was transformed with the resulting product. After the resulting transformant was confirmed by a mini-prep, plasmid DNA was prepared (medium-scale extraction).

### (6) Expression of Tob protein and Growth Experiment in YPH500-12 Strain (ρ⁻)

An YPH500-12 strain (ρ⁻) was transformed simultaneously with the prepared four kinds of plasmids according to the lithium acetate method. The resulting transformant was subcultured on a different CSM-TRP-URA-LUE-HIS solid medium, and cultured at 30°C for 2 to 3 days, and thereby a tryptophan-, uracil-, leucine-, and histidine-requiring transformant was screened. A colony was inoculated from the plate into 5 ml of a CSM-TRP-URA-LUE-HIS Raf (2%) medium, and the colony was pre-cultured. The pre-culture was carried out at 30°C for 72 hours. Thereafter, 100 µl was taken from the culture medium, and added to 900 µl of a CSM-TRP-URA-LUE-HIS Raf (2%) + Gal (2%) medium, thereby diluting the culture medium 10-folds. The amount 40 µl of the diluted culture medium was taken, and added to a microplate, and 160 µl of a CSM-TRP-URA-LUE-HIS Raf (2%) + Gal (2%) medium was added thereto, thereby diluting the diluted culture medium further 5-folds. A microplate containing the culture medium which was diluted 50-folds according to these experimental procedures was cultured in an incubator at 30°C while shaking. The growth was observed by measuring a turbidity of the culture medium using a microplate reader over time. In addition, for the purpose of performing Western blotting, the culture in a test tube was concurrently carried out. A 100 µl culture medium was taken from the pre-culture medium, subcultured on 5 ml of a CSM-TRP-URA-LUE-HIS Raf (2%)+Gal (2%) medium, and cultured in an incubator at 30°C while shaking. The protein for Western blotting was collected at a point where the culture time was 168 hours. The amount 50 µg of the protein was electrophoresed using a 10% acrylamide gel, and then transferred onto a nitrocellulose membrane. Tob antibody (manufactured by Immuno-Biological Laboratories Co., Ltd.), and the myc antibody were used for detecting the Tob protein.

As the control experiment, an experiment was carried out for transformation with only pESC-TRP-*tob* as a plasmid in the same manner as in Example B-1.

### (7) Analysis of Lowering in Growth of YPH500-12 strain (ρ⁻) by Expression of Tob Protein

A proliferation curve of an YPH500-12 strain (r-) was prepared in the same manner as in Example B-1.

However, in this example, a 96-well microplate was used in place of the test tube during the culture, and a turbidity was measured by determining absorbance at 620 nm with a microplate reader (manufactured by Dainippon Pharmaceutical Co., Ltd.) in place of cret-meter.

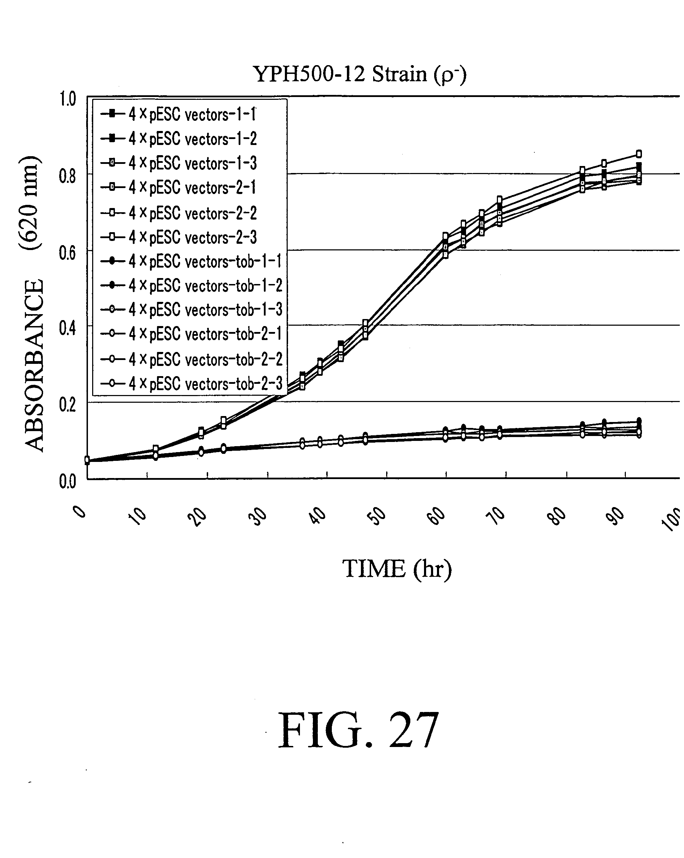

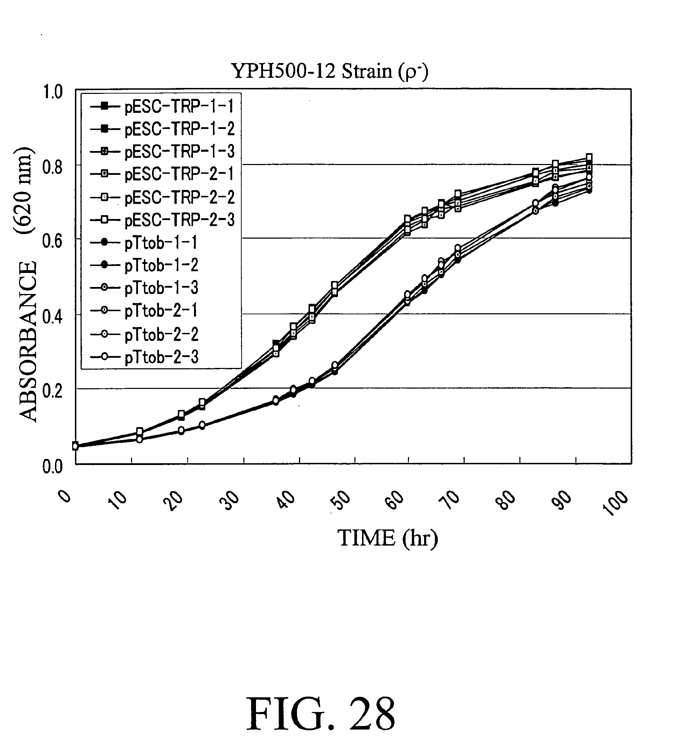

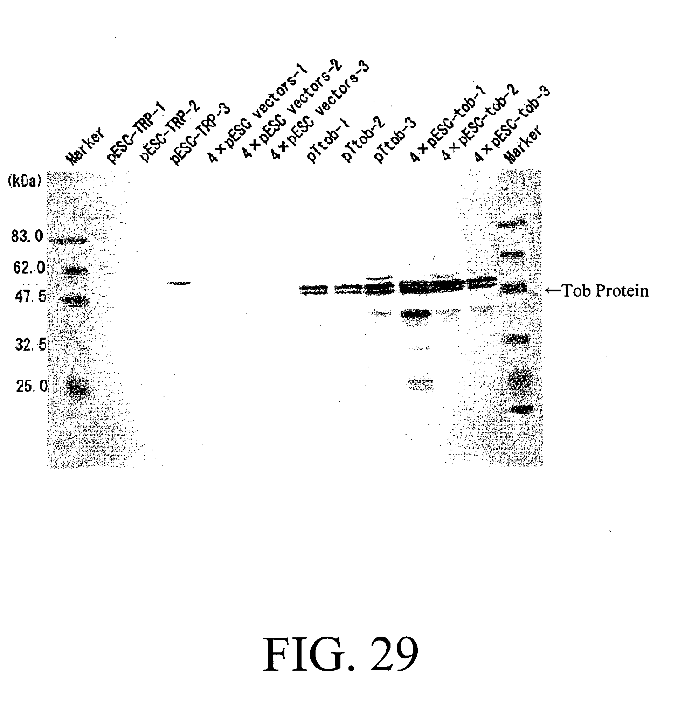

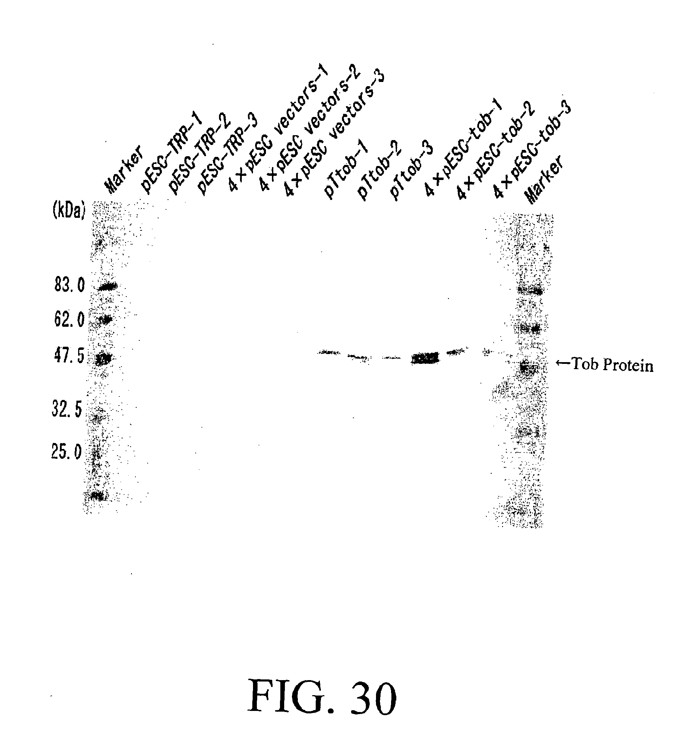

A proliferation curve of an yeast transformed simultaneously with the four kinds of vectors is shown in Figure 27, and a proliferation curve of the control experiment is shown in Figure 28. In addition, the results of Western blotting are shown in Figures 29 and 30.

In the figures, the four kinds of plasmids are collectively abbreviated as 4 × pESC vectors.

It can be seen from Figures 27 and 28 that each of the transformed yeasts generally shows uniform growth without variance in a non-expression state (only a vector was introduced) and in an expression state (one in which *tob* gene-incorporated vector was introduced) of the Tob protein. In addition, it can be seen that the growth is clearly lowered in the expression state of the Tob protein, as compared to the non-expression state of the protein. Further, it can be seen that when the protein was expressed simultaneously using the four kinds of vectors, the degree of the lowering in growth is enhanced. In addition, as shown in Figures 29 and 30, the expressed Tob protein was captured in the yeast in the expression state of Tob protein.

### SEQUENCE LISTING FREE TEXT

SEQ ID NO: 5 is a nucleotide sequence for a primer to amplify *tob* gene.
SEQ ID NO: 6 is a nucleotide sequence for a primer to amplify *tob* gene.
SEQ ID NO: 7 is a nucleotide sequence for a primer to amplify *lck* gene.
SEQ ID NO: 8 is a nucleotide sequence for a primer to amplify *lck* gene.
SEQ ID NO: 9 is a nucleotide sequence for a primer to amplify *lckYF* gene.

### INDUSTRIAL APPLICABILITY

According to the present invention, a method of screening a physiologically active substance capable of efficiently screening a physiologically active substance is provided. The physiologically active substance obtained by the method has, for instance, the similar functions to those of a factor involved in intracellular signaling at a G0/G1 phase in a mammal, or function of inhibiting or enhancing the function. Therefore, there is a possibility that the physiologically active substance serves as a therapeutic agent and/or prophylactic agent for various diseases involved in proliferation and differentiation of cells. In addition, yeast deficient in aspiration ability capable of expressing a heterogeneous protein, used in the method, has the properties that the growing state is changed in the expression state of the protein as compared to that of the non-expression state of the protein, the change is more sensitive as compared to the yeast having aspiration ability capable of expressing a heterogeneous protein. Moreover, the yeast has the property that more stable growth is exhibited in the non-expression state of a heterogeneous protein. The properties greatly contribute to efficiency of screening of the physiologically active substance in the above-mentioned method.

## Claims

1. A method of screening a physiologically active substance, comprising the steps of:
(1) contacting a transformed yeast with a test sample, wherein the transformed yeast is capable of expressing a heterogeneous protein, and shows a change in a growing state in an expression state of the protein as compared to that in a non-expression state of the protein;
(2) culturing the yeast under conditions that the protein is capable of being expressed; and
(3) measuring the growing state of the yeast,
wherein the physiologically active substance is judged to be present in the test sample in a case where the growth of the yeast is lowered or improved in the presence of the test sample as compared to that in the absence of the test sample as a control.

2. The method of screening according to claim 1, wherein the heterogeneous protein is capable of lowering the growth of the yeast as compared to that of the non-expression state.

3. The method of screening according to claim 1 or 2, wherein the heterogeneous protein is a protein involved in regulating cell cycle of a mammal cell.

4. The method of screening according to claim 3, wherein the protein involved in regulating cell cycle of a mammal cell is a protein involved in intracellular signaling of G0/G1 phase of a mammal cell.

5. The method of screening according to any one of claims 1 to 4, wherein the growing state of the yeast is determined in the step (3) by monitoring a change in turbidity of an yeast culture medium, a morphological change of the yeast, a change in wet-weight of the yeast, a change in dry-weight of the yeast, a change in endogenous enzyme activity of the yeast or a change in amount of an endogenous protein of the yeast.

6. The method of screening according to any one of claims 1 to 5, wherein the transformed yeast is deficient in aspiration ability.

7. A transformed yeast being capable of expressing a heterogeneous protein, and showing a change in a growing state in an expression state of the protein as compared to that in a non-expression state of the protein.

8. The transformed yeast according to claim 7, wherein the change in the growing state is lowering of the growth.

9. The transformed yeast according to claim 7 or 8, wherein the heterogeneous protein comprises at least an active site of the protein in an active state.

10. The transformed yeast according to any one of claims 7 to 9, wherein the heterogeneous protein is a protein involved in regulating cell cycle of a mammal cell.

11. The transformed yeast according to claim 10, wherein the protein involved in regulating cell cycle of a mammal cell is a protein involved in intracellular signaling of G0/G1 phase of a mammal cell.

12. The transformed yeast according to claim 11, wherein the protein involved in intracellular signaling of G0/G1 phase of a mammal cell is a protein belonging to Tob family and/or a protein belonging to Caf family.

13. The transformed yeast according to claim 12, wherein the protein belonging to Tob family is:
(a) a protein comprising in an N-terminal region of the amino acid sequence thereof the amino acid sequence of SEQ ID NO: 1;
(b) a protein comprising in an N-terminal region of the amino acid sequence thereof an amino acid sequence having deletion, addition, insertion or substitution of at least one amino acid residue in the amino acid sequence of SEQ ID NO: 1, wherein the protein induces growth inhibition of the transformed yeast in the expression state;
(c) a protein comprising in an N-terminal region of the amino acid sequence thereof an amino acid sequence having a sequence identity of 20% or more to the amino acid sequence of SEQ ID NO: 1, wherein the protein induces growth inhibition of the transformed yeast in the expression state; or
(d) a protein comprising in an N-terminal region of the amino acid sequence thereof an amino acid sequence having a sequence identity of 20% or more to the amino acid sequence of SEQ ID NO: 2 in a region from an N-terminal to 100 amino acid residues, wherein the protein induces growth inhibition of the transformed yeast in the expression state.

14. The transformed yeast according to claim 12, wherein the protein belonging to Caf family is:
(a) a protein comprising the amino acid sequence of SEQ ID NO: 4;
(b) a protein comprising an amino acid sequence having deletion, addition, insertion or substitution of at least one amino acid residue in the amino acid sequence of SEQ ID NO: 4, wherein the protein induces growth inhibition of the transformed yeast in the expression state; or
(c) a protein comprising an amino acid sequence having a sequence identity of 20% or more to the amino acid sequence of SEQ ID NO: 4, wherein the protein induces growth inhibition of the transformed yeast in the expression state.

15. The transformed yeast according to any one of claims 7 to 14, wherein the transformed yeast comprising a gene encoding a heterogeneous protein operably in expression ligated to a promoter.

16. The transformed yeast according to any one of claims 7 to 15, wherein the transformed yeast is deficient of aspiration ability, which is used for the method of screening as defined in claim 6.

17. A physiologically active substance obtainable by the method of screening as defined in any one of claims 1 to 6.

18. A kit for screening a physiologically active substance, comprising the yeast as defined in any one of claims 7 to 16.


SEQUENCE LISTING

<110> Biochemical and Pharmacological Laboratories Inc.

<120> A screening method for physiologically active substance

<130> 03-061-PCT

<160> 9

<210> 1
<211> 138
<212> PRT
<213> Homo sapiens

<400> 1
Met Gln Leu Glu Ile Gln Val Ala Leu Asn Phe Ile Ile Ser Tyr Leu
1               5                   10                  15

Tyr Asn Lys Leu Pro Arg Arg Arg Val Asn Ile Phe Gly Glu Glu Leu
            20                  25                  30

Glu Arg Leu Leu Lys Lys Lys Tyr Glu Gly His Trp Tyr Pro Glu Lys
        35                  40                  45

Pro Tyr Lys Gly Ser Gly Phe Arg Cys Ile His Ile Gly Glu Lys Val
    50                  55                  60

Asp Pro Val Ile Glu Gln Ala Ser Lys Glu Ser Gly Leu Asp Ile Asp
65                  70                  75                  80

Asp Val Arg Gly Asn Leu Pro Gln Asp Leu Ser Val Trp Ile Asp Pro
                85                  90                  95

Phe Glu Val Ser Tyr Gln Ile Gly Glu Lys Gly Pro Val Lys Val Leu
            100                 105                 110

Tyr Val Asp Asp Asn Asn Glu Asn Gly Cys Glu Leu Asp Lys Glu Ile
        115                 120                 125

Lys Asn Ser Phe Asn Pro Glu Ala Gln Val
    130                 135

<210> 2

<211> 19
<212> PRT
<213> Homo sapiens

<400> 2
Tyr Glu Gly His Trp Tyr Pro Glu Lys Pro Tyr Lys Gly Ser Gly Phe
1               5                   10                  15

Arg Cys Ile

<210> 3
<211> 21
<212> PRT
<213> Homo sapiens

<400> 3
Leu Pro Gln Asp Leu Ser Val Trp Ile Asp Pro Phe Glu Val Ser Tyr
1               5                   10                  15

Gln Ile Gly Glu Lys
            20

<210> 4
<211> 285
<212> PRT
<213> Homo sapiens

<400> 4
Met Pro Ala Glu Thr Val Asp His Ser Gln Arg Ile Cys Glu Val Trp
1               5                   10                  15

Ala Cys Asn Leu Asp Glu Glu Met Lys Lys Ile Arg Gln Val Ile Arg
            20                  25                  30

Lys Tyr Asn Tyr Val Ala Met Asp Thr Glu Phe Pro Gly Val Val Ala
        35                  40                  45

Arg Pro Ile Gly Glu Phe Arg Ser Asn Ala Asp Tyr Gln Tyr Gln Leu
    50                  55                  60

Leu Arg Cys Asn Val Asp Leu Leu Lys Ile Ile Gln Leu Gly Leu Thr
65                  70                  75                  80

Phe Met Asn Glu Gln Gly Glu Tyr Pro Pro Gly Thr Ser Thr Trp Gln

                85                  90                  95

Phe Asn Phe Lys Phe Asn Leu Thr Glu Asp Met Tyr Ala Gln Asp Ser
            100                 105                 110

Ile Glu Leu Leu Thr Thr Ser Gly Ile Gln Phe Lys Lys His Glu Glu
        115                 120                 125

Glu Gly Ile Glu Thr Gln Tyr Phe Ala Glu Leu Leu Met Thr Ser Gly
    130                 135                 140

Val Val Leu Cys Glu Gly Val Lys Trp Leu Ser Phe His Ser Gly Tyr
145                 150                 155                 160

Asp Phe Gly Tyr Leu Ile Lys Ile Leu Thr Asn Ser Asn Leu Pro Glu
                165                 170                 175

Glu Glu Leu Asp Phe Phe Glu Ile Leu Arg Leu Phe Phe Pro Val Ile
            180                 185                 190

Tyr Asp Val Lys Tyr Leu Met Lys Ser Cys Lys Asn Leu Lys Gly Gly
        195                 200                 205

Leu Gln Glu Val Ala Glu Gln Leu Glu Leu Glu Arg Ile Gly Pro Gln
    210                 215                 220

His Gln Ala Gly Ser Asp Ser Leu Leu Thr Gly Met Ala Phe Phe Lys
225                 230                 235                 240

Met Arg Glu Met Phe Phe Glu Asp His Ile Asp Asp Ala Lys Tyr Cys
                245                 250                 255

Gly His Leu Tyr Gly Leu Gly Ser Gly Ser Ser Tyr Val Gln Asn Gly
            260                 265                 270

Thr Gly Asn Ala Tyr Glu Glu Glu Ala Asn Lys Gln Ser
275 280

<210> 5
<211> 30
<212> DNA
<213> Artificial Sequence

<220>

<223> PCR primer to amplify tob gene

<400> 5
cccggatcca tgcagcttga aatccaagta

<210> 6
<211> 30
<212> DNA
<213> Artificial Sequence

<220>
<223> PCR primer to amplify tob gene

<400> 6
cccgtcgacg ttagccataa caggctggaa

<210> 7
<211> 21
<212> DNA
<213> Artificial Sequence

<220>
<223> PCR primer to amplify lck gene

<400> 7
atgggctgtg gctgcagctc a

<210> 8
<211> 30
<212> DNA
<213> Artificial Sequence

<220>
<223> PCR primer to amplify lck gene

<400> 8
cccgtcgaca ggctgaggct ggtactggcc

<210> 9
<211> 30
<212> DNA
<213> Artificial Sequence

<220>
<223> PCR primer to amplify lckYF gene

<400> 9
cccgtcgaca ggctgaggct gaaactggcc